(19) Europäisches Patentamt
European Patent Office
Office européen des brevets

(11) EP 1 915 375 B1

(12) **FASCICULE DE BREVET EUROPEEN**

(45) Date de publication et mention de la délivrance du brevet:
**07.10.2009 Bulletin 2009/41**

(21) Numéro de dépôt: **06794298.7**

(22) Date de dépôt: **07.08.2006**

(51) Int Cl.:
***C07D 471/18*** (2006.01) ***A61K 31/439*** (2006.01)
***A61P 25/00*** (2006.01)

(86) Numéro de dépôt international:
**PCT/FR2006/001912**

(87) Numéro de publication internationale:
**WO 2007/020344 (22.02.2007 Gazette 2007/08)**

(54) **DÉRIVÉS DE -PYRIDINYL-i-AZABICYCLO[3.2.1]OCTANE, A LEUR PRÉPARATION ET LEUR APPLICATION EN THÉRAPEUTIQUE**

5-PYRIDINYL-1-AZABICYCLO[3.2.1]OCTAN-DERIVATE, HERSTELLUNGSVERFAHREN UND DEREN VERWENDUNG FÜR THERAPEUTIKA

DERIVATIVES OF 5-PYRIDINYL-1-AZABICYCLO[3.2.1]OCTANE, PREPARATION METHOD THEREOF AND USE OF SAME IN THERAPEUTICS

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK RS**

(30) Priorité: **12.08.2005 FR 0508528**

(43) Date de publication de la demande:
**30.04.2008 Bulletin 2008/18**

(73) Titulaire: **Sanofi-Aventis**
**75013 Paris (FR)**

(72) Inventeurs:
- **GALLI, Frédéric**
**F-92420 Vaucresson (FR)**
- **LECLERC, Odile**
**F-91300 Massy (FR)**
- **LOCHEAD, Alistair**
**F-94220 Charenton le Pont (FR)**
- **VACHE, Julien**
**F-75011 Paris (FR)**

(74) Mandataire: **Gaslonde, Aude et al**
**Sanofi-Aventis**
**Département Brevets**
**174, Avenue de France**
**75013 Paris (FR)**

(56) Documents cités:
**EP-A- 0 664 293** **WO-A-98/54181**
**WO-A-03/057697** **US-A- 5 817 679**

- **HOLLADAY ET AL: "Neuronal Nicotinic Acetylcholine Receptros as Targets for Drug Discovery" 19 décembre 1997 (1997-12-19), JOURNAL OF MEDICINAL CHEMISTRY, AMERICAN CHEMICAL SOCIETY. WASHINGTON, US, PAGE(S) 4169-4194 , XP002124847 ISSN: 0022-2623 page 4180; figure 5; exemples 43-54**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 915 375 B1

## Description

La présente invention se rapporte à des dérivés de 5-pyridinyl-1-azabicyclo[3.2.1]octane, à leur préparation et à leur application en thérapeutique.

On connaît déjà des composés dérivés de 5-pyridinyl-1-azabicyclo[3.2.1]octane, décrits dans le document WO03/057697 affins in vitro pour les récepteurs nicotiniques du type $\alpha_4\beta_2$ et $\alpha_7$.
Il existe toujours une nécessité de trouver et de développer des produits affins pour les récepteurs nicotiniques et sélectifs vis-à-vis des récepteurs nicotiniques contenant la sous-unité $\alpha_7$.
L'invention répond à ce but en proposant des composés nouveaux, qui présentent une bonne affinité pour les récepteurs nicotiniques et une bonne sélectivité vis-à-vis des récepteurs nicotiniques contenant la sous-unité $\alpha_7$.

La présente invention a pour objet les composés répondant à la formule générale (I)

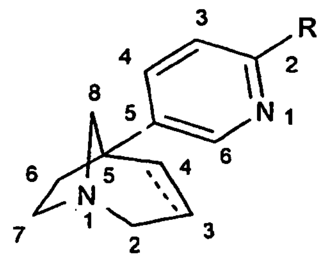

**(I)**

dans laquelle :

R représente un groupe choisi parmi un pyrazolyle, imidazolyle, triazolyle, oxazolyle, oxadiazolyle, thiazolyle, isothiazolyle, thiadiazolyle, tétrazolyle, ce groupe pouvant éventuellement être substitué par un ou plusieurs groupes choisis parmi les atomes d'halogènes, les groupes $(C_1-C_6)$alkyle, $(C_1-C_6)$alcoxy, trifluorométhoxy, trifluorométhyle, nitro, cyano, hydroxy, amino, $(C_1-C_6)$alkylamino ou di$(C_1-C_6)$alkylamino ;
la liaison carbone-carbone entre les positions 3 et 4 du cycle azabicyclooctane est simple ou double.

Par ailleurs, l'atome de carbone en position 5 du cycle azabicyclo[3.2.1]octane est asymétrique, de sorte que les composés de l'invention peuvent exister sous forme de deux énantiomères ou de mélange de ces derniers. Ces énantiomères ainsi que leurs mélanges, y compris les mélanges racémiques, font partie de l'invention.

Les composés de formule (I) peuvent également exister à l'état de bases ou de sels d'addition à des acides. De tels sels d'addition font partie de l'invention.
Ces sels peuvent être préparés avec des acides pharmaceutiquement acceptables, mais les sels d'autres acides utiles, par exemple pour la purification ou l'isolement des composés de formule (I), font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de la présente invention, on entend par :

- un atome d'halogène : un atome de fluor, de chlore, de brome ou d'iode ;
- un groupe alkyle : un groupe aliphatique saturé linéaire ou ramifié. A titre d'exemples, on peut citer les groupes méthyle, éthyle, propyle, isopropyle, butyle, isobutyle, tertbutyle, pentyle, etc ;
- un groupe alcoxy : un radical -O-alkyle dont le groupe alkyle est tel que précédemment défini.

Parmi les composés de formule (I) objets de l'invention, un premier sous-groupe de composés est constitué par les composés pour lesquels :

R représente un groupe choisi parmi un pyrazolyle, imidazolyle, triazolyle, oxazolyle, oxadiazolyle, thiazolyle, isothiazolyle, thiadiazolyle, tétrazblyle, ce groupe pouvant éventuellement être substitué par un ou plusieurs groupes $(C_1-C_6)$alkyle, plus particulièrement méthyle ; et
la liaison carbone-carbone entre les positions 3 et 4 du cycle azabicyclooctane est simple ou double.

Parmi les composés de formule (I) objets de l'invention, un deuxième sous-groupe de composés est constitué

par les composés pour lesquels :

R représente un groupe choisi parmi un pyrazolyle, imidazolyle, triazolyle, oxazolyle, oxadiazolyle, thiazolyle, isothiazolyle, thiadiazolyle, tétrazolyle, ce groupe pouvant éventuellement être substitué par un ou plusieurs groupes ($C_1$-$C_6$)alkyle, plus particulièrement méthyle; isobutyle ou *n*-propyle ; et
la liaison carbone-carbone entre les positions 3 et 4 du cycle azabicyclooctane est simple ou double.

Parmi les composés de formule (I) objets de l'invention, un troisième sous-groupe de composés est constitué par les composés pour lesquels :

R représente un groupe choisi parmi un pyrazolyle, imidazolyle, triazolyle, oxazolyle, oxadiazolyle, thiazolyle, tétrazolyle, ce groupe pouvant éventuellement être substitué par un ou plusieurs groupes choisis parmi les atomes d'halogènes, les groupes ($C_1$-$C_6$)alkyle, ($C_1$-$C_6$)alcoxy, trifluorométhoxy, trifluorométhyle, nitro, cyano, hydroxy, amino, ($C_1$-$C_6$)alkylamino ou di($C_1$-$C_6$)alkylamino ; et
la liaison carbone-carbone entre les positions 3 et 4 du cycle azabicyclooctane est simple ou double.

Parmi les composés de formule (I) objets de l'invention, un quatrième sous-groupe de composés est constitué par les composés pour lesquels :

R représente un groupe choisi parmi un pyrazolyle, imidazolyle, triazolyle, oxazolyle, oxadiazolyle, thiazolyle, tétrazolyle, ce groupe pouvant éventuellement être substitué par un ou plusieurs groupes ($C_1$-$C_6$)alkyle, plus particulièrement méthyle ; et
la liaison carbone-carbone entre les positions 3 et 4 du cycle azabicyclooctane est simple ou double.

Parmi les composés de formule (I) objets de l'invention, un cinquième sous-groupe de composés est constitué par les composés pour lesquels :

R représente un groupe choisi parmi un pyrazolyle, imidazolyle, triazolyle, oxazolyle, oxadiazolyle, thiazolyle, tétrazolyle, ce groupe pouvant éventuellement être substitué par un ou plusieurs groupes ($C_1$-$C_6$)alkyle, plus particulièrement méthyle, isobutyle ou *n*-propyle ; et
la liaison carbone-carbone entre les positions 3 et 4 du cycle azabicyclooctane est simple ou double.

Parmi les composés de formule (I) objets de l'invention, un sixième sous-groupe de composés est constitué par les composés pour lesquels :

R représente un groupe pyrazolyle éventuellement substitué par un ou plusieurs groupes ($C_1$-$C_6$)alkyle, ($C_1$-$C_6$) alcoxy, trifluorométhoxy, trifluorométhyle, nitro, cyano, hydroxy, amino, ($C_1$-$C_6$)alkylamino ou di($C_1$-$C_6$)alkylamino ;
la liaison carbone-carbone entre les positions 3 et 4 du cycle azabicyclooctane est simple ou double.

Parmi les composés de formule (I) objets de l'invention, un septième sous-groupe de composés est constitué par les composés pour lesquels :

R représente un groupe pyrazolyle éventuellement substitué par un ou plusieurs groupes ($C_1$-$C_6$)alkyle, plus particulièrement méthyle, isobutyle ou *n*-propyle ;
la liaison carbone-carbone entre les positions 3 et 4 du cycle azabicyclooctane est simple ou double.

Parmi les composés de formule (I) objets de l'invention, un huitième sous-groupe de composés est constitué par les composés pour lesquels :

R représente un groupe pyrazolyle éventuellement substitué par un ou plusieurs groupes ($C_1$-$C_6$)alkyle, plus particulièrement méthyle, isobutyle ou *n*-propyle ;
la liaison carbone-carbone entre les positions 3 et 4 du cycle azabicyclooctane est simple.

Parmi les composés de formule (I) objets de l'invention, on peut citer les composés de formule :

- 5-[2-(1-méthyl-1*H*-pyrazol-4-yl)pyridin-5-yl]-1-azabicyclo[3.2.1]-octane ;
- 5-[2-(1*H*-pyrazol-4-yl)pyridin-5-yl]-1-azabicyclo[3.2.1]-octane ;
- 5-[2-(1*H*-pyrazol-4-yl)pyridin-5-yl]-1-azabicyclo[3.2.1]-oct-3-ène ;

- 5-[2-(1-méthyl-1*H*-pyrazol-4-yl)pyridin-5-yl]-1-azabicyclo[3.2.1]-oct-3-ène ;
- 5-[2-(-1*H*-imidazol-1-yl)pyridin-5-yl]-1-azabicyclo[3.2.1]oct-3-ène
- 5-[2-(-1*H* imidazol-4-yl)pyridin-5-yl]-1-azabicyclo[3.2.1]oct-3-ène
- 5-[2-(-1*H*-imidazol-4-yl)pyridin-5-yl]-1-azabicyclo[3.2.1]octane
- 5-[2-(-1*H*-imidazol-1-yl)pyridin-5-yl]-1-azabicyclo[3.2.1]octane
-5-[2-(-1*H*-imidazol-2-yl)pyridin-5-yl]-1-azabicyclo[3.2.1]octane
- 5-[2-(3,5-diméthyl-1*H*-pyrazol-4-yl)pyridin-5-yl]-1-azabicyclo[3.2.1]-octane
- 5-[2-(1*H*-1,2,4-triazol-3-yl)pyridin-5-yl]-1-azabicyclo[3.2.1]-octane ;
- 5-[2-(5-méthyl-1,2,4-oxadiazol-3-yl)pyridin-5-yl]-1-azabicyclo[3.2.1]-octane ;
- 5-[2-(1,3-oxazol-2-yl)pyridin-5-yl]-1-azabicyclo[3.2.1]-octane ;
- 5-[2-(thiazol-4-yl)pyridin-5-yl]-1-azabicyclo[3.2.1]octane
- 5-[2-(pyrazol-3-yl)pyridin-5-yl]-1-azabicyclo[3.2.1]octane
- 5-[2-(2-méthyl-thiazol-5-yl)pyridin-5-yl]-1-azabicyclo[3.2.1]octane
- 5-12-(tétrazol-5-yl)pyridin-5-yl]-1-azabicyclo[3.2.1]-octane;
-5-[2-(1-isobutyl-1*H*-pyrazol-4-yl)pyridin-5-yl]-1-azabicyclo[3.2.1]-octane ;
- 5-[2-(1-*n*-propyl-1*H*-pyrazol-4-yl)pyridin-5-yl]-1-azabicyclo[3.2.1]-octane;

à l'état de base ou de sel d'addition à un acide, ainsi qu'à l'état d'hydrate ou de solvat ;
à l'état d'énantiomère pur ou de mélange d'énantiomères.

Dans ce qui suit, on entend par groupe protecteur un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans « Protective Groups in Organic Synthesis », Green et al., 2nd Edition (John Wiley & Sons, Inc., New York), 1991.

On entend par groupe partant, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un méthanesulfonate, benzènesulfonate, p-toluènesulfonate, triflate, acétate, etc. Des exemples de groupes partants ainsi que des références pour leur préparation sont donnés dans « Advances in Organic Chemistry », J. March, 3rd Edition, Wiley Interscience, 1985, p. 310-316.

Les composés de formule générale (I) peuvent être préparés par un procédé illustré selon le schéma 1 suivant. On effectue une réaction d'addition sur l'anion lithié d'un composé hétérocyclique de formule générale (III), dans laquelle Z représente un atome de brome et W représente un atome d'halogène, en présence du 3-oxo-1-azabicyclo[2.2.2] octane, de formule (II). L'anion lithié du composé hétérocyclique de formule générale (III) est obtenu par échange halogène - métal avec un dérivé alkyllithium.

On obtient le composé de formule (IV) qui lorsqu'il est traité en milieu acide à chaud conduit au composé de formule (V). L'hydrogénation catalytique de la double liaison conduit au composé de formule (VI). Les composés de formule générale (I), dans laquelle la liaison carbone-carbone entre les positions 3 et 4 du cycle azabicyclooctane est double et R représente un groupe pyrazolyle, imidazolyle, triazolyle, oxazolyle, oxadiazolyl, thiazolyle, isothiazolyle, thiadiazolyle ou tétrazolyle éventuellement substitués sont obtenus à partir du composé de formule (V) dans laquelle Z représente un atome de brome.

Schéma 1

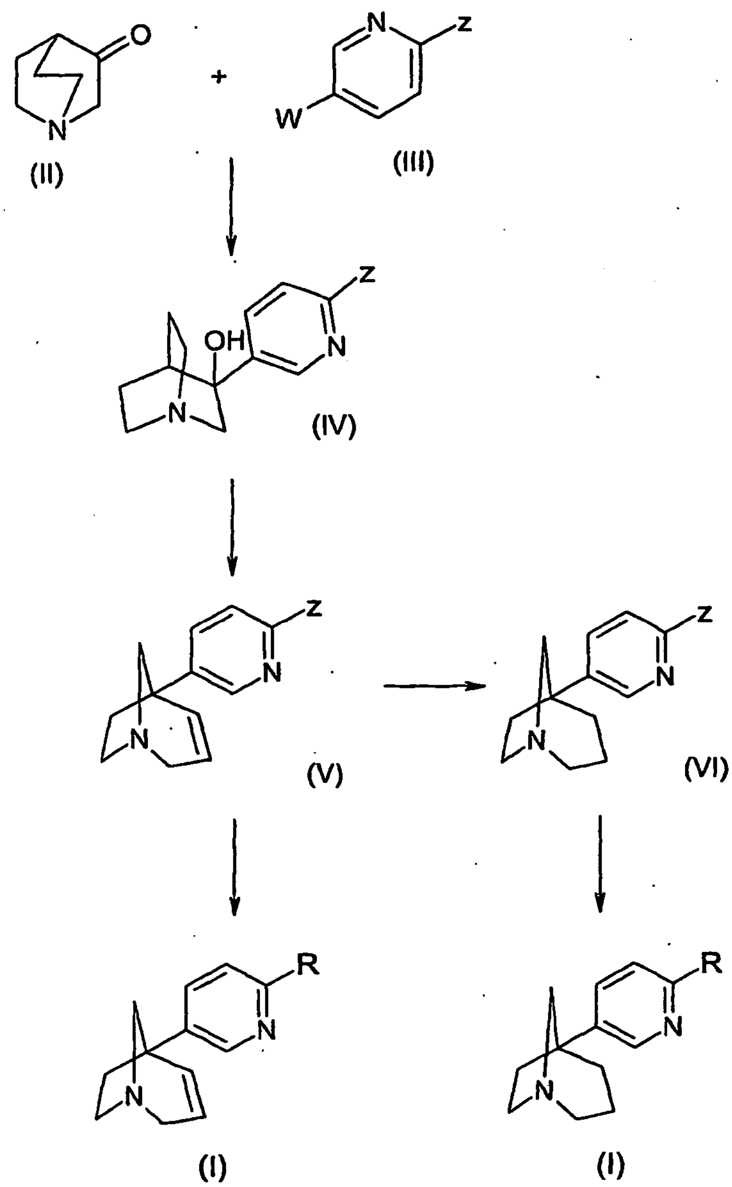

(II) (III) (IV) (V) (VI) (I) (I)

Les composés de formule générale (I), dans laquelle la liaison carbone-carbone entre les positions 3 et 4 du cycle azabicyclooctane est simple et R représente un groupe pyrazolyle, imidazolyle, triazolyle, oxazolyle, oxadiazolyle, thiazolyle, isothiazolyle, thiadiazolyle ou tétrazolyle éventuellement substitué, sont obtenus à partir du composé de formule (VI) dans laquelle Z représente un atome de brome.

Le substituant R peut ainsi être introduit sur le composé de formule (V) ou (VI) dans laquelle Z représente un atome de brome selon toutes méthodes connues de l'homme du métier, telles que par exemple:

- avec un acide boronique de formule $R\text{-}B(OH)_2$ dans laquelle R est tel que défini dans la formule générale (I), en présence d'un catalyseur au palladium, par exemple le tétrakistriphénylphosphine palladium ;
- avec un composé de formule R-H dans laquelle R est tel que défini dans la formule générale (I), en présence d'une base forte, par exemple l'hydrure de sodium, dans un solvant, par exemple le diméthylformamide ;
- avec un dérivé stanneux de formule $R\text{-}Sn[(CH_2)_3CH_3)]_3$ dans laquelle R est tel que défini dans la formule générale (I), en présence d'un catalyseur au palladium, par exemple le bis(triphénylphosphino)dichloropalladium ;
- avec un composé de formule R-H dans laquelle R est tel que défini dans la formule générale (I), en présence de

n-butyllithium, de chlorure de zinc et d'un catalyseur au palladium, par exemple le tétrakistriphénylphosphine palladium.

Les composés de formule générale (I) dans laquelle la liaison carbone-carbone entre les positions 3 et 4 du cycle azabicyclooctane est simple et R représente un groupement triazolyle, oxadiazolyle ou tétrazolyle éventuellement substitué peuvent également être préparés à partir du composé de formule (VII).

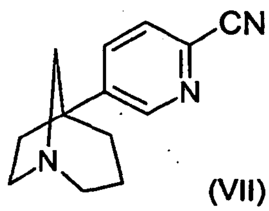
(VII)

Par exemple :

- lorsque R représente un groupement triazolyle, le composé de formule (I) peut être obtenu à partir du composé de formule (VII) en présence d'une base forte, telle qu'une solution de méthylate de sodium, et d'hydrazide formique dans un solvant tel que le méthanol.
- lorsque R représente un groupement oxadiazolyle, le composé de formule (I) peut être obtenu en deux étapes à partir du composé de formule (VII),
transformé tout d'abord en N-hydroxy-carboxamidine de formule (VIII)

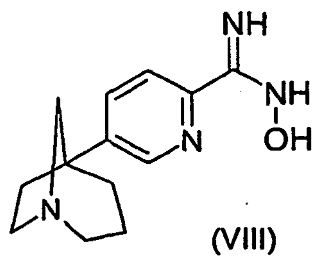
(VIII)

par exemple en présence de chlorhydrate d'hydroxylamine en milieu basique, le composé de formule (VIII) ainsi obtenu réagissant ensuite avec l'anhydride acétique dans un solvant tel que la pyridine pour donner le composé de formule (I) attendu.
- lorsque R représente un groupement tétrazolyle, le composé de formule (I) peut être obtenu à partir du composé de formule (VII) en présence d'azoture de sodium et de chlorure d'ammonium dans un solvant tel que le diméthylformamide.

Le composé de formule (VII) est préparé à partir du composé de formule (VI), dans laquelle Z représente un atome de brome, en présence par exemple de cyanure de potassium et de tétrakis(triphénylphosphino)-palladium dans un solvant tel que le diméthylformamide.
La préparation du composé de formule (V) dans laquelle R représente un atome de brome est décrit dans WO 03057697.
Le 3-oxo-1-azabicyclo[2.2.2]octane de formule (II) est disponible dans le commerce.
Les composés de formule générale (III) sont disponibles dans le commerce ou sont accessibles par des méthodes décrites dans la littérature.
Dans le schéma 1, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'homme du métier.
L'invention, selon un autre de ses aspects, a également pour objet les composés de formules (IV), (V), (VI), (VII) et (VIII). Ces composés sont utiles comme intermédiaires de synthèse des composés de formule générale (I).
Les exemples suivants décrivent la préparation de certains composés conformes à l'invention. Ces exemples ne sont pas limitatifs et ne font qu'illustrer la présente invention. Les numéros des composés donnés entre parenthèses dans les titres renvoient à ceux donnés dans la première colonne du tableau ci-après, qui illustre les structures chimiques

et les propriétés physiques de quelques composés selon l'invention.

**<u>Exemple 1</u> (composé N°1)**

(-)-Bromhydrate (2 : 1) de 5-[2-(1-méthyl-1*H*-pyrazol-4-yl)pyridin-5-yl]-1-azabicyclo[3.2.1]-octane

1.1 bromhydrate (1 :1) de 5-(2-bromopyridin-5-yl)-1-azabicyclo[3.2.1]octane

Dans un flacon d'hydrogénation, on introduit 1,95 g (7,354 mmoles) de 5-(2-bromopyridin-5-yl)-1-azabicyclo [3.2.1]oct-3-ène (WO03057697) dans 40 ml de méthanol, puis 195 mg d'oxyde de platine sont mis en suspension. Le milieu est agité à température ambiante sous une pression d'hydrogène de 26 psi pendant 45 min. On filtre le milieu réactionnel sur terre de diatomées et on élimine le solvant par évaporation sous pression réduite. On purifie le résidu obtenu par chromatographie sur colonne de gel de silice en éluant par un mélange de chloroforme, méthanol et ammoniaque dans les proportions 95/5/0,5. On obtient 1,4 g de 5-(2-bromopyridin-5-yl)-1-azabicyclo[3.2.1]octane sous forme d'huile cireuse.
Le sel de bromhydrate (1 :1) du 5-(2-bromopyridin-5-yl)-1-azabicyclo[3.2.1]octane est ensuite obtenu par addition d'1 équivalent d'une solution 5,7 N d'acide bromhydrique dans l'acide acétique. On obtient 1,82 g de produit attendu.

1.2 (+) et (-)-bromhydrates (1 :1) de 5-(2-bromopyridin-5-yl)-1-azabicyclo[3.2.1]octane

Le mélange racémique de bromhydrate (1:1) de 5-(2-bromopyridin-5-yl)-1-azabicyclo[3.2.1]octane, obtenu à l'étape 1.1, est dédoublé par chromatographie liquide sur support chiral de façon à obtenir les énantiomères dextrogyre et lévogyre, respectivement, le (+)-bromhydrate (1 :1) de 5-(2-bromopyridin-5-yl)-1-azabicyclo[3.2.1]octane et le (-)-bromhydrate (1:1) de 5-(2-bromopyridin-5-yl)-1 azabicyclo[3.2.1]octane.

(+)-Bromhydrate (1 : 1) de 5-(2-bromopyridin-5-yl)-1-azabicycto[3.2.1]octane : $[\alpha_D^{20}] = +\ 24,4°$ (c = 1, $CH_3OH$) (-)-Bromhydrate (1 : 1) de 5-(2-bromopyridin-5-yl)-1-azabicyclo[3.2.1]octane: $[\alpha_D^{20}] = -\ 23,1°$ (c= 1, $CH_3OH$)

1.3 (-)-Bromhydrate (2 : 1) de 5-[2-(1-méthyl-1*H*-pyrazol-4-yl)pyridin-5-yl]-1-aza-bicyclo-[3.2.1]-octane

Dans un ballon tricol de 10 ml on introduit successivement 0,162 g (0,78 mmole) d'acide 1-méthyl-4-pyrazolylboronique, 0,160 g (0,6 mmole) de (-)-5-(2-bromopyridin-5-yl)-1 azabicyclo[3.2.1]octane (obtenu par réaction du (-)-bromhydrate (1 :1) de 5-(2-bromopyridin-5-yl)-1-azabicyclo[3.2.1]octane, préparé à l'étape 1.2, avec une solution aqueuse saturée de carbonate de sodium), en solution dans 3 ml de toluène et 0,3 ml d'éthanol. On ajoute ensuite 0,035 g (0,03 mmole) de tétrakis(triphénylphosphino)palladium et 0,6 ml d'une solution aqueuse 2M de carbonate de sodium et on chauffe le mélange à 105°C pendant 18 h. On le refroidit à température ambiante, on évapore le solvant sous pression réduite et on reprend le résidu dans 10 ml de chloroforme et on filtre sur terre de diatomées. On concentre le solvant sous pression réduite et on purifie le résidu par chromatographie sur colonne de gel de silice en éluant par un mélange de chloroforme, méthanol et ammoniaque dans les proportions 95/5/0,5. On obtient 0,15 g de (-)-5-[2-(1-méthyl-1*H*-pyrazol-4-yl)pyridin-5-yl]-1-aza-bicyclo-[3.2.1]-octane que l'on dissout dans 2 ml d'alcool isopropylique pour ajouter 0,196 ml d'une solution d'acide bromhydrique 5,7N dans l'acide acétique. On collecte les cristaux obtenus par filtration et on les sèche sous vide.
On obtient 0,163 g de produit.
Point de fusion : 259-261°C.
RMN $^1H$ (DMSO) δ (ppm): 8.50 (1H, s); 8.45 (1H, s); 8.15 (1H, s); 8.10 (1H, d); 7.95 (1H, d); 4.00 (3H, s); 3.85-3.10 (6H, m); 2.50-1.80 (6H, m). $[\alpha_D^{20}] = -24,4°$ (c = 1, $CH_3OH$)

Le composé n°2 a été préparé selon la méthode décrite dans l'exemple 1.
Les composés 3 et 4 ont été préparés selon la méthode décrite dans l'exemple 1, à partir de 5-(2-bromopyridin-5-yl)-1-azabicyclo[3.2.1]oct-3-ène (WO03057697).
Les composés n°12, 13 et 15 ont été préparés selon la méthode décrite dans l'exemple 1, à partir de (+)-5-(2-bromopyridin-5-yl)-1-azabicyclo[3.2.1]octane, obtenu selon l'étape 1.2.

**Exemple 2 (composé N°24)**

(+)-(S,S)-Dibenzoyl tartrate (1 :1) de 5-[2-(1-méthyl-1*H*-pyrazol-4-yl)pyridin-5-yl]-1-aza-bicyclo-[3.2.1]-octane

2.1 (+/-)-5-[2-(1-méthyl-1H pyrazol-4-yl)pyridin-5-yt]-1-aza-bicyclo-[3.2.1]-octane (composé N°28)

Dans un ballon tricol de 250 ml on introduit successivement 7,08 g (34,06 mmoles) d'acide 1-méthyl-4-pyrazolylboronique, 7,0 g (26,20 mmoles) de (+/-)-5-(2-bromopyridin-5-yl)-1-azabicyclo[3.2.1]octane, obtenu selon l'étape 1.1 de l'exemple 1, en solution dans 140 ml de toluène et 14 ml d'éthanol. On ajoute ensuite 1,82 g (1,57 mmole) de tétrakis(triphénylphosphino)palladium et 26,20 ml (52,40 mmoles) d'une solution aqueuse 2M de carbonate de sodium et on chauffe le mélange à 90°C pendant 12 h. Le milieu réactionnel est refroidi à température ambiante, versé dans 50 ml d'eau, extrait deux fois par du chloroforme et les phases organiques réunies sont séchées sur sulfate de sodium, filtrées et évaporées sous vide. Le résidu obtenu est purifié par chromatographie sur colonne de gel de silice en éluant par un mélange de chloroforme, méthanol et ammoniaque dans les proportions 95/5/0,5. On obtient 5,30 g de produit attendu sous forme d'une poudre jaune pâle.
Point de fusion : 138-140°C.
RMN $^1$H (DMSO) δ (ppm): 8.37 (1 H, d); 8.19 (1H, s); 7.90 (1 H, s); 7.58 (1 H, dd); 7.50 (1H, dd); 3.87 (3H, s); 3.09-2.60 (6H, m); 2.21-1.31 (6H, m).

2.2 (+)-(S,S)-Dibenzoyl tartrate (1:1) de 5-[2-(1-méthyl-1*H* pyrazol-4-yl)pyridin-5-yl]-1-aza-bicyclo-[3.2.1]-octane

Dans un ballon de 250 ml on dissout 9,70 g (36,15 mmoles) de (+/-)-5-[2-(1-méthyl-1H pyrazol-4-yl)pyridin-5-yl]-1-aza-bicydo-[3.2.1]-octane, obtenu à l'étape 2.1, dans 100 ml d'éthanol, on additionne 13,08 g (36,51 mmoles) d'acide (S,S)-dibenzoyl tartrique, le milieu réactionnel est agité à température ambiante pendant 5 minutes et concentré sous pression réduite.
Le solide résultant est dissout dans 50 ml d'éthanol puis chauffé au reflux jusqu'à dissolution totale. Le milieu est lentement ramené à température ambiante. Les cristaux obtenus sont filtrés puis séchés sous vide pour conduire à 7,10 g du composé désiré avec une pureté optique de 95,4%. Ces derniers sont recristallisés dans les conditions décrites ci-dessus, dans 25 ml d'éthanol pour conduire à 5,54 g de (+)-(S,S)-Dibenzoyl tartrate (1:1) de 5-[2-(1-méthyl-1*H*-pymzol-4-yl)pyridin-5-yl]-1-aza-bicyclo-[3.2.1]-octane sous forme de cristaux blancs avec une pureté optique de 98,8%.

Point de fusion : 156-158°C. $[\alpha_D^{20}] = +78,5°$ (c = 0,558, $CH_3OH$)

RMN $^1$H (DMSO) δ (ppm): 8.39 (s, 1H); 8.22 (s, 1H); 7.92 (s, 1H); 7.90-7.78 (m, 4H); 7.65-7.35 (m, 8H); 5.60 (s, 2H); 3.86 (s, 3H); 3.62-2.96 (m, 8H); 2.54-1.65 (m, 6H).

**Exemple 3 (composé N°5)**

Bromhydrate (1 : 1) de 5-[2-(-1*H*-imidazol-1-yl)pyridin-5-yl]-1-azabicyclo[3.2.1]oct-3-ène

Dans un ballon tricol de 10 ml on introduit 0,64 g (9,42 mmoles) d'imidazole en solution dans 3 ml de diméthylformamide. On ajoute ensuite 0,415 g (10,4 mmoles) d'hydrure de sodium en dispersion à 60% dans l'huile et on agite à température ambiante pendant 1 heure. Le mélange est alors additionné à une solution de 5-(2-bromopyridin-5-yl)-1-azabicyclo[3.2.1]oct-3-ène (WO0305769) (0,5 g, 1,89 mmole) dans la diméthylformamide et le milieu réactionnel est chauffé à 85°C pendant 15 heures puis à 110°C pendant 24 heures et le solvant est évaporé sous pression réduite. Le résidu est repris dans 10 ml de chloroforme et 10 ml d'une solution aqueuse saturée de carbonate de sodium. La phase aqueuse est extraite à nouveau par 10 ml de chloroforme et les phases organiques réunies sont lavées par une solution aqueuse saturée de chlorure de sodium, séchées sur sulfate de sodium, filtrées et concentrées sous pression réduite. Le résidu est purifié par chromatographie sur colonne de gel de silice en éluant par un mélange de chloroforme, méthanol et ammoniaque dans les proportions 95/5/0,5. On obtient 0,235 g de 5-[2-(-1*H*-imidazol-1-yl)pyridin-5-yl]-1-azabicyclo[3.2.1]oct-3-ène que l'on dissout dans 3 ml d'alcool isopropylique pour additionner 0,327 ml d'une solution d'acide bromhydrique 5,7N dans l'acide acétique. Les cristaux formés sont collectés par filtration et séchés sous vide.
Point de fusion : 233-235°C
RMN $^1$H (DMSO) δ (ppm): 8.55 (1H, s); 8.50 (1 H, s); 8.05 (1 H, d); 7.95 (1H, s); 7.85 (1 H, d); 7.15 (1H, s); 6.15 (1H, d); 5.75 (1H, dt); 4.20-4.10 (1H, d); 4.00-3.40 (5H, m); 2.80-2.60 (1 H, t); 2.45-2.30 (1 H, t).

Le composé n°8 a été préparé selon la méthode décrite dans l'exemple 3, à partir de (-)-5-(2-bromopyridin-5-yl)-1-azabicyclo[3.2.1]octane, obtenu selon l'étape 1.2 de l'exemple 1.
Le composé n°11 a été préparé selon la méthode décrite dans l'exemple 3, à partir de (+)-5-(2-bromopyridin-5-yl)-1-

azabicyclo[3.2.1]octane, obtenu selon l'étape 1.2 de l'exemple 1.

**Exemple 4 (composé N°6)**

Chlorhydrate (3 : 1) de 5-[2-(-1*H*-imidazol-4-yl)pyridin-5-yl]-1-azabicyclo[3.2.1]oct-3-ène

4.1 5-[2-(-1-triphénylméthyl-imidazol-4-yl)pyridin-5-yl]-1-azabicyclo[3.2.1]oct-3-ène

Dans un ballon tricol de 10 ml on introduit successivement 0,25 g (0,94 mmole) de 5-(2-bromopyridin-5-yl)-1-azabicyclo[3.2.1]oct-3-ène (WO0305769) en solution dans 3 ml de tétrahydrofuranne, 1,24 g (2,07 mmoles) de 1-triphénylméthyl-4-tributylstannyl imidazole et 0,06 g (0,08 mmole) de bis(triphénylphosphino)dichloropalladium. Le mélange est alors chauffé à 85°C pendant 15 heures puis dilué dans 10 ml de chloroforme et 10 ml d'une solution aqueuse saturée de carbonate de sodium. La phase aqueuse est extraite à nouveau par 10 ml de chloroforme et les phases organiques réunies sont lavées par une solution aqueuse saturée de chlorure de sodium, séchées sur sulfate de sodium, filtrées et concentrées sous pression réduite. Le résidu est purifié par chromatographie sur colonne de gel de silice en éluant par un mélange de chloroforme, méthanol et ammoniaque dans les proportions 97/3/0,3. on obtient 0,36 g de produit attendu sous forme de solide amorphe.

4.2 Chlorhydrate (3 : 1) de 5-[2-(-1*H*-imidazol-4-yl)pyridin-5-yl]-1-azabicyclo[3.2.1]oct-3-ène

Dans un ballon tricol de 10 ml on introduit 0,36 g (0,733 mmole) de 5-[2-(-1-triphénylméthyl-imidazol-4-yl) pyridin-5-yl]-1-azabicyclo[3.2.1]oct-3-ène, obtenu à l'étape 4.1, en solution dans 4 ml de méthanol. On ajoute ensuite 0,8 ml d'une solution d'acide chlorhydrique 6N dans l'alcool isopropylique et on chauffe le milieu réactionnel à 80°C pendant 3 heures. Le solvant est concentré sous pression réduite et le résidu est trituré dans l'éther diéthytique. Les cristaux obtenus sont collectés par filtration et séchés sous vide.
Point de fusion : 306-308°C
RMN $^1$H (DMSO) δ (ppm): 11.95 (1H, s); 9.20 (1H, s); 8.65 (1H, s); 8.40 (1H, s); 8.10 (1H, d); 8.00. (1H, d); 6.20 (1H, d); 5.75 (1 H, dt); 4.15 (1 H, d); 3.95-3.35 (5H, m); 2.80-2.60 (1 H, t); 2.45-2.30 (1 H, t).

Le composé n°7 a été préparé selon la méthode décrite dans l'exemple 4, à partir de (-)-5-(2-bromopyridin-5-yl)-1-azabicyclo[3.2.1]octane, obtenu selon l'étape 1.2 de l'exemple 1.
Le composé n°9 a été préparé selon la méthode décrite dans l'exemple 4, à partir de (+)-5-(2-bromopyridin-5-yl)-1-azabicyclo[3.2.1]octane, obtenu selon l'étape 1.2 de l'exemple 1.

**Exemple 5 (composé N°10)**

(-)-Chlorhydrate (2 : 1) de 5-[2-(-1*H*-imidazol-2-yl)pyridin-5-yl]-1-azabicyclo[3.2.1]octane

5.1 (-)-5-[2-(-1*H*-imidazol-2-yl)pyridin-5-yl]-1-azabicyclo[3.2.1]octane

Dans un tricol de 25ml on introduit 1 g (5,7 mmoles) de 1-(diméthylaminosulfonyl)imidazole en solution dans 9 ml de tétrahydrofuranne. Le milieu réactionnel est refroidi à -78°C et on ajoute 4 ml d'une solution 1,6M de n-butyllithium dans l'hexane goutte à goutte en 20 minutes. On ajoute ensuite 0,73g (5,4 mmoles) de chlorure de zinc en solution dans 4 ml de tétrahydrofuranne. On agite en laissant la température remonter jusqu'à 20°C puis on ajoute successivement 1,5g (11,1 mmoles) de chlorure de zinc, 0,1g (0,09 mmole) de tetrakis(triphénylphosphino)palladium et 0,56g (2,1 mmoles) de (-)-5-(2-bromopyridin-5-yl)-1-azabicyclo[3.2.1]octane (préparé selon la méthode décrite à l'étape 1.2 de l'exemple 1), en solution dans 5 ml de tétrahydrofuranne. Le mélange est alors chauffé au reflux pendant 24 heures puis refroidi à température ambiante. On ajoute 30 ml d'une solution aqueuse d'hydroxyde de sodium à 30% et 50 ml de chloroforme. La phase aqueuse est extraite par du chloroforme puis les phases organiques réunies sont lavées par une solution aqueuse saturée de chlorure de sodium, séchées sur sulfate de sodium, filtrées et concentrées sous pression réduite. Le résidu est purifié par chromatographie sur colonne de gel de silice en éluant par un mélange chloroforme, méthanol et ammoniaque dans les proportions 95/5/0,5 et utilisé tel que dans l'étape suivante.

5.2 (-)-Chlorhydrate (2 : 1) de 5-[2-(-1*H*-imidazol-2-yl)pyridin-5-yl]-1-azabicyclo[3.2.1]-octane

Le produit obtenu à l'étape 5.1 est mis en solution dans 10 ml de dioxanne et 1,5 ml d'une solution aqueuse d'acide chlorhydrique 2N. Le milieu est agité à température ambiante pendant 2 heures puis le solvant est évaporé sous pression réduite. Le résidu est repris dans 30 ml de chloroforme et 30 ml d'une solution aqueuse saturée de carbonate de sodium. La phase aqueuse est extraite par du chloroforme et les phases organiques réunies sont séchées sur sulfate

de sodium, filtrées et concentrées sous pression réduite. Le résidu obtenu est mis en solution dans 5ml d'alcool isopropylique et traité avec un excès d'acide chlorhydrique en solution dans l'alcool isopropylique. Les cristaux obtenus sont collectés par filtration et séchés sous vide. On obtient 0,23g de produit.
Point de fusion : 231-233°C
RMN $^1$H (DMSO) δ (ppm): 11.45 (1H, s); 8.75 (1H, s); 8.55 (1H, d); 8.05 (1H, d); 7.80 (2H, s); 3.75 (1H, d); 3.60-3.10 (5H, m); 2.55-1.75 (6H, m). $[\alpha_D^{20}] = -34,2°$ (c = 0,26, $CH_3OH$)

Le composé n°19 a été préparé selon la méthode décrite dans l'exemple 5.
Le composé n°20 a été préparé selon la méthode décrite à l'étape 5.1 de l'exemple 5 suivie d'une étape de déprotection en milieu basique, en présence d'une solution isovolume d'hydroxyde de sodium aqueux à 35% et de dioxanne, à température ambiante.
Les composés n°18 et 21 ont été préparés selon la méthode décrite à l'étape 5.1 de l'exemple 5.
Le composé n°14 a été préparé selon la méthode décrite dans l'exemple 5, à partir de (+)-5-(2-bromopyridin-5-yl)-1-azabicyclo[3.2.1]octane, obtenu selon l'étape 1.2 de l'exemple 1.

**Exemple 6 (composé N°16)**

(-)-Bromhydrate (2 : 1) de 5-[2-(1*H*-1,2,4-triazol-3-yl)pyridin-5-yl]-1-azabicyclo[3.2.1]-octane

- 6.1 (-)-(1-azabicyclo[3.2.1]oct-5-yl)pyridine-2-carbonitrile

Dans un réacteur de 25 ml on introduit successivement 1,5 g (4,3 mmoles) de bromhydrate de (-)-5-(2-bromopyridin-5-yl)-1-azabicyclo[3.2.1]octane (préparé selon la méthode décrite à l'étape 1.2 de l'exemple 1), en solution dans 12 ml de diméthylformamide, 0,42 g (6,46 mmoles) de cyanure de potassium et 5 g (4,3 mmoles) de tétrakis(triphénylphosphino)-palladium. Le mélange est ensuite chauffé à 90°C pendant 3 heures puis neutralisé par une solution aqueuse saturée de carbonate de sodium. La phase aqueuse est extraite par du chloroforme et les phases organiques réunies sont séchées sur sulfate de sodium, filtrées et concentrées sous pression réduite. Le résidu est purifié par chromatographie sur colonne de gel de silice en éluant par un mélange de chloroforme, méthanol et ammoniaque dans les proportions 95/5/0,5. on obtient ainsi 0,705 g de produit attendu sous forme de solide amorphe.

6.2 (-)-Bromhydrate (2 : 1) de 5-[2-(1*H*-1,2,4-triazol-3-yl)pyridin-5-yl]-1-azabicyclo-[3.2.1]-octane

Dans un réacteur de 10 ml on introduit 0,22 g (1,03 mmole) de (-)-(1-azabicyclo[3.2.1]oct-5-yl)pyridine-2-carbonitrile, obtenu à l'étape 6.1, en solution dans 3 ml de méthanol. On purge à l'argon et on ajoute ensuite 0,04 ml (0,26 mmole) d'une solution 5,25 N de méthylate de sodium dans le méthanol et, après 15 minutes d'agitation à température ambiante, on ajoute 0,065 g (1,08 mmole) d'hydrazide formique. Le milieu est ensuite chauffé à 85°C pendant 24 heures et le solvant est évaporé. Le résidu est purifié par chromatographie sur colonne de gel de silice en éluant par un mélange de chloroforme, méthanol et ammoniaque dans les proportions 85/15/1,5. On obtient ainsi 0,155g de (-)-5-[2-(1*H*-1,2,4-triazol-3-yl)pyridin-5-yl]-1-azabicyclo-[3.2.1]-octane que l'on traite par 0,19 ml d'une solution 5,7N d'acide bromhydrique dans l'acide acétique. Les cristaux obtenus sont collectés par filtration et séchés sous vide.
Point de fusion : 215-217°C
RMN $^1$H (DMSO) δ (ppm): 10.15 (1H, s); 8.65 (1H, s); 8.40 (1H, s); 8.10 (1 H, d); 7.95 (1 H, d); 3.80-3.10 (6H, m); 2.30-1.75 (6H, m). $[\alpha_D^{20}] = -19,9°$ (c = 1, $CH_3OH$)

Le composé n°22 a été préparé selon la méthode décrite dans l'exemple 6, à partir de (+)-(1-azabicyclo[3.2.1] oct-5-yl)pyridine-2-carbonitrile, lui-même obtenu à partir (+)-5-(2-bromopyridin-5-yl)-1-azabicyclo[3.2.1]octane (préparé selon la méthode décrite à l'étape 1.2 de l'exemple 1).

**Exemple 7 (composé N°17)**

(-)-Bromhydrate (1 : 1) de 5-[2-(5-methyl-1,2,4-oxadiazol-3-yl)pyridin-5-yl]-1-azabicyclo-[3.2.1]octane

7.1 (-)-5-(1-azabicyclo[3.2.1]oct-5-yl)-*N*-hydroxy-pyridine-2-carboxamidine

Dans un réacteur de 10 ml on introduit successivement 0,3 g (1,4 mmole) de (-)-(1-azabicyclo[3.2.1]oct-5-yl) pyridine-2-carbonitrile, préparé selon la méthode décrite à l'étape 6.1 de l'exemple 5, 0,39 g (5,63 mmoles) de chlorhydrate d'hydroxylamine, 0,78 g (5,65 mmoles) de carbonate de potassium et 5 ml d'alcool éthylique. Le mélange est

chauffé au reflux pendant 3 heures puis filtré. Le filtrat est concentré sous pression réduite, le résidu repris dans 10 ml d'eau. La phase aqueuse est extraite par du chloroforme et les phases organiques réunies sont lavées par une solution aqueuse saturée de chlorure de sodium, séchées sur sulfate de sodium et évaporées sous vide. On obtient ainsi 0,29 g de produit sous forme de solide amorphe.

7.2 (-)-Bromhydrate (1:1) de 5-[2-(5-méthyl-1,2,4-oxadiazol-3-yl)pyridin-5-yl]-1-azabi-cyclo[3.2.1]octane

Dans un réacteur de 10 ml on introduit 0,28 g (1,14 mole) de (-)-5-(azabicyclo[3.2.1]oct-5-yl)-*N*-hydroxypyridine-2-carboximidamide, préparé à l'étape 7.1, en solution dans 10 ml de pyridine. On ajoute ensuite 0,1 ml (1,13 mmole) d'anhydride acétique et le milieu est agité à température ambiante pendant 15 heures puis chauffé à 110°C pendant 5 heures. Le solvant est concentré sous pression réduite et le résidu est repris par une solution aqueuse saturée de carbonate de sodium. La phase aqueuse est extraite par du chloroforme et les phases organiques réunies sont séchées sur sulfate de sodium, filtrées et évaporées sous vide. Le résidu est purifié par chromatographie sur colonne de gel de silice en éluant par un mélange de chloroforme, méthanol et ammoniaque dans les proportions 96/4/0,4. On obtient ainsi 0,077g de (-)-5-[2-(5-méthyl-1,2,4-oxadiazol-3-yl)pyridin-5-yl]-1-azabicyclo[3.2.1]octane que l'on solubilise dans 1 ml d'alcool isopropylique pour ajouter 0,05 ml d'une solution d'acide bromhydrique 5,7N dans l'acide acétique. Les cristaux obtenus sont collectés par filtration et séchés sous pression réduite.
Point de fusion : 321-323°C
RMN $^1$H (DMSO) δ (ppm): 8.70 (1H, s); 8.05 (1H, d); 7.95 (1 H, d); 3.80-3.20 (6H, m); 3.70 (3H, s); 2.35-1.75 (6H, m).

$[\alpha_D^{20}]$ **= - 26,4°** (c = 1, $CH_3OH$)

**<u>Exemple 8</u> (composé N°23)**

(+)-5-[2-(2*H*-tétrazol-5-yl)pyridin-5-yl]-1-azabicyclo[3.2.1]octane

Dans un réacteur de 10 ml on introduit successivement 0,350 g (4,64 mmoles) de (+)-(1-azabicyclo[3.2.1]oct-5-yl)pyridine-2-carbonitrile (préparé selon une méthode analogue à celle décrite à l'étape 6.1 de l'exemple 6 à partir de (+)-5-(2-bromopyridin-5-yl)-1-azabicyclo[3.2.1]octane obtenu à l'étape 1.2 de l'exemple 1), 0,117 g (1,80 mmole) d'azoture de sodium, 0,022 g (0,4 mmole) de chlorure d'ammonium et 2 ml de diméthylformamide. Le mélange est ensuite chauffé à 80°C pendant 15 heures puis le solvant est évaporé sous pression réduite. Le résidu est repris dans le méthanol à température ambiante. L'insoluble résultant est filtré et le filtrat est concentré sous pression réduite. Le résidu est chromatographié sur colonne de gel de silice en éluant par un mélange de chloroforme, méthanol et ammoniaque dans les proportions 80/20/2. On obtient ainsi 0,368 g de produit sous forme de cristaux.
Point de fusion : 319-321 °C
RMN $^1$H (DMSO) δ (ppm): 8.45 (1H, s); 7.95 (1H, d); 7.70 (1H, d); 3.60-3.10 (6H, m); 2.45-1.70 (6H, m).

$[\alpha_D^{20}]$ **= + 25,2°** (c = 0,06, DMSO)

Le tableau 1 qui suit illustre les structures chimiques et les propriétés physiques de quelques exemples de composés selon l'invention. Dans ce tableau :

- dans la colonne "DL", "=" signifie que la liaison carbone-carbone entre les positions 3 et 4 du cycle azabicyclooctane est double , et "-" signifie que la liaison carbone-carbone entre les positions 3 et 4 du cycle azabicyclooctane est simple.
- dans la colonne "Sel", "-" désigne un composé à l'état de base, "HBr" désigne un bromhydrate et "HCl" désigne un chlorhydrate. Les rapports molaires acide:base sont indiqués en regard ;
- dans la colonne $[\alpha_D^{20}]$ ($CH_3OH$), la valeur indiquée représente le pouvoir rotatoire du composé, la concentration en g/100ml dans le méthanol à laquelle cette mesure a été réalisée étant indiquée entre parenthèses ; les composés sans mention dans cette colonne sont des racémates.

Tableau 1

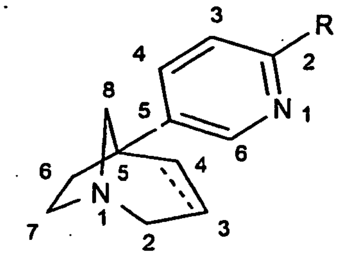

(I)

| N° | DL | R | Sel | $[\alpha_D^{20}]$ ($CH_3OH$) | PF (°C) (Point de fusion) |
|---|---|---|---|---|---|
| 1 | - | 1-$CH_3$-4-pyrazolyl | HBr 2:1 | - 24,4 (c=1) | 259-261 |
| 2 | - | 4-pyrazolyl | HBr 1:1 | - | 293-295 |
| 3 | = | 4-pyrazolyl | HBr 2:1 | - | 322-324 |
| 4 | = | 1-$CH_3$-4-pyrazolyl | HBr 2:1 | - | 294-296 |
| 5 | = | 1-imidazolyl | HBr 1:1 | - | 233-235 |
| 6 | = | 4-imidazolyl | HCl 3:1 | - | 306-308 |
| 7 | - | 4-imidazolyl | HCl 3:1 | -24,8 (c=0,8) | 292-294 |
| 8 | - | 1-imidazolyl | HBr 1:1 | - 14,2 (c = 0,9) | 191-193 |
| 9 | - | 4-imidazolyl | HCl 2:1 | +23,6 (c = 1) | 288-290 |
| 10 | - | 2-imidazolyl | HCl 2:1 | - 34,2 (c = 0,26) | 231-233 |
| 11 | - | 1-imidazolyl | HBr 1:1 | + 22,2 (c = 1) | 233-235 |
| 12 | - | 4-pyrazolyl | HBr 2:1 | + 23,6 (c = 1) | 285-287 |
| 13 | - | 1-$CH_3$-4-pyrazolyl | HBr 2:1 | + 22,1 (c = 1) | 247-249 |
| 14 | - | 2-imidazolyl | HBr 2:1 | + 1 8,3 (c = 1) | 234-236 |
| 15 | - | 3,5-$(CH_3)_2$-4-pyrazolyl | HBr 2:1 | +19,6(c=1) | 321-323 |
| 16 | - | 3-(1,2,4-triazolyl) | HBr 2:1 | -19,9(c=1) | 215-217 |
| 17 | - | 3-(5-$CH_3$-1,2,4-oxadiazolyl) | HBr 1:1 | - 26,4 (c = 1) | 321-323 |
| 18 | - | 2-(1,3-oxazolyl) | - | - 37,5 (c = 0,4) | 115-117 |
| 19 | - | 4-thiazolyl | HBr1:1 | - 27,7 (c = 1) | 272-274 |
| 20 | - | 3-pyrazolyl | HBr 1:1 | + 23,8 (c = 0,38) | 273-275 |
| 21 | - | 2-$CH_3$-5-thiazolyl | HBr 1:1 | - 23,2 (c = 0,36) | 267-269 |
| 22 | - | 3-(1,2,4-triazolyl) | HBr 2:1 | + 20,5 (c = 1) | 215-217 |
| 23 | - | 5-tétrazolyl | - | + 25,2 (c = 0,06)* | 319-321 |
| 24 | - | 1-$CH_3$-4-pyrazolyl | (S,S)-Dibenzoyl tartrate 1:1 | +78,5 (c = 0,558) | 156-158 |
| 25 | - | 1-$CH_3$-4-pyrazolyl | - | +36,1 (c = 0,49) | 142-144 |
| 26 | - | 1-$CH_3$-4-pyrazolyl | Fumarate 1:1 | + 17,4 (c = 0,55) | 200-202 |
| 27 | - | 1-$CH_3$-4-pyrazolyl | HCl 2:1 | + 29,4 (c = 0,48) | 191-193 |

(suite)

| N° | DL | R | Sel | $[\alpha_D^{20}]$ ($CH_3OH$) | PF (°C) (Point de fusion) |
|---|---|---|---|---|---|
| **28** | - | 1-$CH_3$-4-pyrazolyl | - | - | 138-140 |
| **29** | - | 1-isobutyl-4-pyrazolyl | HBr 2:1 | - 24,7 (c = 1) | 104-106 |
| **30** | - | 1-*n*-propyl-4-pyrazolyl | HBr 2:1 | - 21,8 (c = 1) | 190-192 |
| * solvant : DMSO | | | | | |

Les composés de l'invention ont fait l'objet d'essais pharmacologiques qui ont mis en évidence leur intérêt comme substances actives de médicaments.

Ainsi ils ont été étudiés quant à leur affinité vis-à-vis des récepteurs nicotiniques contenant la sous unité $\alpha_7$, selon les méthodes décrites par Mark et Collins dans J. Pharmacol. Exp. Ther. 1982, 22, 564 et par Marks et coll. dans Mol. Pharmacol. 1986, 30, 427.

On décapite des rats mâles OFA de 150 à 200 g, on prélève rapidement la totalité du cerveau, on l'homogénéise à l'aide d'un broyeur Polytron™ dans 15 volumes d'une solution de sucrose à 0,32 M à 4 °C, puis on le centrifuge à 1000 G pendant 10 min. On élimine le culot et on centrifuge le surnageant à 8000 G pendant 20 min à 4 °C. On récupère le culot et on l'homogénéise à l'aide d'un broyeur Polytron™ dans 15 volumes d'eau bidistillée à 4°C, puis on le centrifuge à 8000 G pendant 20 min. On élimine le culot et on centrifuge le surnageant et la couche de peau ("buffy coat") à 40000 G pendant 20 min. On récupère le culot, on le remet en suspension avec 15 volumes d'eau bidistillée à 4°C et on le centrifuge encore une fois à 40000 G pendant 20 min avant de le conserver à -80°C.

Le jour de l'expérience on décongèle lentement le tissu et on le met en suspension dans 5 volumes de tampon. On préincube 150 µl de cette suspension membranaire à 37°C pendant 30 min, à l'obscurité, en présence ou en absence du composé à tester. Puis les membranes sont incubées pendant 60 min à 37°C, à l'obscurité, en présence de 50 µl de [$^3$H]-$\alpha$-bungarotoxine à 1 nM dans un volume final de 250 µl) de tampon HEPES 20 mM, polyéthylènimine 0,05%. On arrête la réaction par filtration sur des filtres Whatman GF/C™ préalablement traités pendant 3 h avec de la polyéthylènimine à 0,05%. On rince les filtres avec deux fois 5 ml de tampon à 4°C et on mesure la radioactivité retenue sur chaque filtre par scintigraphie liquide. On détermine la liaison non spécifique en présence de $\alpha$-bungarotoxine à 1 µM finale ; la liaison non spécifique représente environ 60 % de la liaison totale récupérée sur le filtre. Pour chaque concentration de composé étudié on détermine le pourcentage d'inhibition de la liaison spécifique de [$^3$H]-$\alpha$-bungarotoxine, puis on calcule la $CI_{50}$, concentration de composé qui inhibe 50% de la liaison spécifique.

Les $CI_{50}$ des composés de l'invention les plus affins se situent entre 0,001 et 1 µM.

Les composés de l'invention ont aussi été étudiés quant à leur affinité vis à vis des récepteurs nicotiniques contenant la sous-unité $\alpha_4\beta_2$ selon les méthodes décrites par Anderson et Americ dans Eur. J. Pharmacol. 1994, 253, 261 et par Hall et coll. dans Brain Res. 1993, 600, 127.

On décapite des rats mâles Sprague Dawley de 150 à 200 g et on prélève rapidement la totalité du cerveau, on l'homogénéise dans 15 volumes d'une solution de sucrose à 0,32 M à 4°C, puis on le centrifuge à 1000 G pendant 10 min. On élimine le culot et centrifuge le surnageant à 20000 G pendant 20 min à 4°C. On récupère le culot et on l'homogénéise à l'aide d'un broyeur Polytron™ dans 15 volumes d'eau bidistillée à 4°C, puis on le centrifuge à 8000 G pendant 20 min. On élimine le culot et on centrifuge le surnageant et la couche de peau (buffy coat) à 40000 G pendant 20 min, on récupère le culot, on le remet en suspension dans 15 ml d'eau bidistillée et on le centrifuge encore une fois à 40000 G avant de le conserver à -80°C.

Le jour de l'expérience on décongèle lentement le tissu et on le met en suspension dans 3 volumes de tampon. On fait incuber 150 µl de cette suspension membranaire à 4°C pendant 120 min en présence de 100 µl de [$^3$H]-cytisine à 1 nM dans un volume final de 500 µl de tampon, en présence ou en absence de composé à tester. On arrête la réaction par filtration sur des filtres Whatman GF/B™ préalablement traités avec de la polyéthylènimine, on rince les filtres avec deux fois 5 ml de tampon à 4°C, et on mesure la radioactivité retenue sur le filtre par scintigraphie liquide. On détermine la liaison non spécifique en présence de (-)-nicotine à 10 µM; la liaison non spécifique représente 75 à 85% de la liaison totale récupérée sur le filtre. Pour chaque concentration de composé étudié on détermine le pourcentage d'inhibition de la liaison spécifique de [$^3$H]-cytisine, à des doses de 1 µM et 10 µM. Pour les composés les plus affins de l'invention, on calcule la $CI_{50}$, concentration de composé qui inhibe 50% de la liaison spécifique.

Les $CI_{50}$ des composés de l'invention les plus affins se situent entre 0,2 et 10 µM.

Les données expérimentales de quelques composés spécifiques sont indiquées dans le tableau 2 qui suit.

**Tableau 2**

| Composé N° | $CI_{50}$ $\alpha_7$ (µM) | Pourcentage d'inhibition de la liaison spécifique de [$^3$H]-cytisine à la dose de 1 µM, pour la sous unité $\alpha_4\beta_2$ (%) |
|---|---|---|
| 11 | 0,083 | 36 |
| 14 | 0,099 | 45 |
| 9 | 0,3 | 24 |

Les composés de l'invention ont également été étudiés quant à leur affinité vis-à-vis des récepteurs nicotiniques périphériques de type ganglionnaire selon la méthode décrite par Houghtling et coll. dans Mol. Pharmacol. 1995, 48, 280. On décongèle des glandes surrénales de boeuf conservées à -80°C, et on les homogénéise à l'aide d'un broyeur Polytron™ dans 20 volumes de tampon Tris-HCl 50 mM à pH 7,4 et à 4°C, puis on les centrifuge à 35000 G pendant 10 min. On élimine le surnageant et on remet le culot en suspension dans 30 volumes de tampon Tris-HCl 50 mM à 4°C et on ré-homogénéise avant de re-centrifuger à 35000 G pendant 10 min. On reprend le dernier culot dans 10 volumes de tampon Tris-HCl à 4°C. On fait incuber 100 µl de membrane soit 10 mg de tissu frais à 24°C pendant 3h en présence de 50 µl de [$^3$H]-epibatidine 0,66 nM finale dans un volume final de 250 µl de tampon, en présence où en absence de composé à tester. On arrête la réaction par dilution des échantillons avec du tampon Tris-HCl 50 µM pH 7,4 à 4°C puis on filtre sur des filtres Whatman GF/C™ préalablement traités pendant 3 heures avec de la polyéthylènimine à 0,5%. On rince les filtres deux fois avec 5 ml de tampon et on mesure la radioactivité retenue sur le filtre par scintigraphie liquide. On détermine la liaison non spécifique en présence de (-)-nicotine 2 mM finale; la liaison non spécifique représente 30 à 40% de la liaison totale récupérée sur le filtre. Pour chaque concentration de produit étudié on détermine le pourcentage d'inhibition de la liaison spécifique de [$^3$H]-epibatidine, puis on calcule la $CI_{50}$, concentration de composé qui inhibe 50% de la liaison spécifique.

Les $CI_{50}$ des composés de l'invention se situent entre 1 et 10 µM.

Les résultats obtenus montrent que certains composés de l'invention sont des ligands sélectifs pour la sous unité $\alpha_7$ du récepteur nicotinique et que d'autres sont mixtes $\alpha_4\beta_2$ et $\alpha_7$.

Ces résultats suggèrent l'utilisation des composés dans le traitement ou la prévention des désordres liés à un dysfonctionnement des récepteurs nicotiniques, notamment au niveau du système nerveux central.

Ces désordres comprennent les altérations cognitives, plus spécifiquement mnésiques (acquisition, consolidation et rappel), mais également les atteintes des processus attentionnels, et les troubles des fonctions exécutives liées à la maladie d'Alzheimer, au vieillissement pathologique (Age Associated Memory Impairment,AAMI) ou normal (senile dementia), au syndrome Parkinsonien, à la trisomie 21 (Down's syndrome), aux pathologies psychiatriques (en particulier les troubles cognitifs associés à la schizophrénie), au syndrome alcoolique de Korsakoff, aux démences vasculaires (multi-infarct dementia, MDI), aux traumatismes crâniens.

Les composés de l'invention pourraient également être utiles dans le traitement des troubles moteurs observés dans la maladie de Parkinson ou d'autres maladies neurologiques telles que la chorée de Huntington, le syndrome de Tourette, la dyskinésie tardive et l'hyperkinésie.

Ils peuvent aussi présenter une activité thérapeutique neuro-protectrice vis à vis des atteintes anatomo-histopathologiques liées aux maladies neuro-dégénératives susnommées.

Les composés de l'invention peuvent également constituer un traitement curatif ou symptomatique des accidents vasculaires cérébraux et des épisodes hypoxiques cérébraux. Ils peuvent être utilisés dans les cas de pathologies psychiatriques : schizophrénie (symptômes positifs et/ou négatifs), troubles bipolaires, dépression, anxiété, attaques de panique, troubles de l'attention avec hyperactivité, comportements compulsifs et obsessionnels.

Ils peuvent prévenir les symptômes dus au sevrage au tabac, à l'alcool, aux différentes substances induisant une dépendance, telles que cocaïne, LSD, cannabis; benzodiazépines.

Ils peuvent être utiles dans le traitement de la douleur d'origine diverse (y compris les douleurs chroniques, neuropathiques ou inflammatoires).

Par ailleurs les composés de l'invention peuvent être utilisés pour le traitement de l'ischémie des membres inférieurs, de l'artérite oblitérante des membres inférieurs (PAD : peripheral arterial disease), de l'ischémie cardiaque (angor stable), de l'infarctus du myocarde, de l'insuffisance cardiaque, du déficit de cicatrisation cutanée des patients diabétiques, des ulcères variqueux de l'insuffisance veineuse.

Les composés de l'invention peuvent aussi être utilisés pour le traitement des processus inflammatoires d'origines diverses, en particulier les inflammations concernant le système nerveux central.

Les composés selon l'invention peuvent donc être utilisés pour la préparation de médicaments, en particulier de médicaments utiles dans le traitement ou la prévention des désordres liés à un dysfonctionnement des récepteurs

nicotiniques, notamment des désordres ci-dessus mentionnés.

Ainsi, selon un autre de ses aspects, l'invention a pour objet des médicaments qui comprennent un composé de formule (I), ou un sel d'addition de ce dernier à un acide pharmaceutiquement acceptable, ou encore un hydrate ou un solvat du composé de formule (I).

Ces médicaments trouvent leur emploi en thérapeutique, notamment dans le traitement ou la prévention des désordres liés à un dysfonctionnement des récepteurs nicotiniques, notamment des désordres ci-dessus mentionnés.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un hydrate ou solvat dudit composé, ainsi qu'au moins un excipient pharmaceutiquement acceptable. Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique; transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention. | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,01 à 20 mg de principe actif par kg de poids corporel, selon la forme galénique.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables ou hydrates ou solvats.

## Revendications

1. Composé répondant à la formule (I)

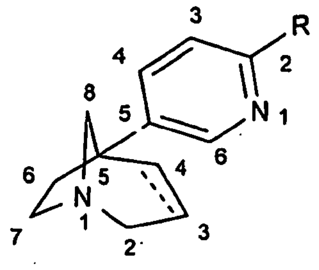

(I)

dans laquelle :

R représente un groupe choisi parmi un pyrazolyle, imidazolyle, triazolyle, oxazolyle, oxadiazolyle, thiazolyle, isothiazolyle, thiadiazolyle, tétrazolyle, ce groupe pouvant éventuellement être substitué par un ou plusieurs groupes choisis parmi les atomes d'halogènes, les groupes ($C_1$-$C_6$)alkyle, ($C_1$-$C_6$)alcoxy, trifluorométhoxy, trifluorométhyle,
nitro, cyano, hydroxy, amino, ($C_1$-$C_6$)alkylamino ou di($C_1$-$C_6$)alkylamino ;
la liaison carbone-carbone entre les positions 3 et 4 du cycle azabicyclooctane est simple ou double ;
à l'état de base ou de sel d'addition à un acide, ainsi qu'à l'état d'hydrate ou de solvat.

**2.** Composé de formule (I) selon la revendication 1, **caractérisé en ce que** R représente un groupe choisi parmi un pyrazolyle, imidazolyle, triazolyle, oxazolyle, oxadiazolyle, thiazolyle, isothiazolyle, thiadiazolyle, tétrazolyle, ce groupe pouvant éventuellement être substitué par un ou plusieurs groupes ($C_1$-$C_6$)alkyle ; et la liaison carbone-carbone entre les positions 3 et 4 du cycle azabicyctooctane est simple ou double; à l'état de base ou de sel d'addition à un acide, ainsi qu'à l'état d'hydrate ou de solvat.

**3.** Composé de formule (I) selon la revendication 1 , **caractérisé en ce que** R représente un groupe choisi parmi un pyrazolyle, imidazolyle, triazolyle, oxazolyle, oxadiazolyle, thiazolyle, tétrazolyle, ce groupe pouvant éventuellement être substitué par un ou plusieurs groupes choisis parmi les atomes d'halogènes, les groupes ($C_1$-$C_6$)alkyle, ($C_1$-$C_6$)alcoxy, trifluorométhoxy, trifluorométhyle, nitro, cyano, hydroxy, amino, ($C_1$-$C_6$)alkylamino ou di($C_1$-$C_6$)alkylamino ; et
la liaison carbone-carbone entre les positions 3 et 4 du cycle azabicyclooctane est simple ou double ;
à l'état de base ou de sel d'addition à un acide, ainsi qu'à l'état d'hydrate ou de solvat.

**4.** Composé de formule (I) selon la revendication 1 ou 3, **caractérisé en ce que** R représente un groupe choisi parmi un pyrazolyle, imidazolyle, triazolyle, oxazolyle, oxadiazolyle, thiazolyle, tétrazolyle, ce groupe pouvant éventuellement être substitué par un ou plusieurs groupes ($C_1$-$C_6$)alkyle ; et
la liaison carbone-carbone entre les positions 3 et 4 du cycle azabicyclooctane est simple ou double ;
à l'état de base ou de sel d'addition à un acide, ainsi qu'à l'état d'hydrate ou de solvat.

**5.** Composé de formule (I) selon la revendication 1 ou 2, **caractérisé en ce que** R représente un groupe pyrazolyle éventuellement substitué par un ou plusieurs groupes ($C_1$-$C_6$)alkyle, ($C_1$-$C_6$) alcoxy, trifluorométhoxy, trifluorométhyle, nitro, cyano, hydroxy, amino, ($C_1$-$C_6$)alkylamino ou di($C_1$-$C_6$)alkylamino;
la liaison carbone-carbone entre les positions 3 et 4 du cycle azabicyclooctane est simple ou double ;
à l'état de base ou de sel d'addition à un acide, ainsi qu'à l'état d'hydrate ou de solvat.

**6.** Composé de formule (I) selon l'une quelconque des revendications 1 à 5, **caractérisé en ce que** R représente un groupe pyrazolyle éventuellement substitué par un ou plusieurs groupes ($C_1$-$C_6$)alkyle ;
la liaison carbone-carbone entre les positions 3 et 4 du cycle azabicyclooctane est simple ou double ;
à l'état de base ou de sel d'addition à un acide, ainsi qu'à l'état d'hydrate ou de solvat.

**7.** Composé de formule (I) selon l'une quelconque des revendications 1 à 6, **caractérisé en ce que** R représente un groupe pyrazolyle éventuellement substitué par un ou plusieurs groupes ($C_1$-$C_6$)alkyle;
la liaison carbone-carbone entre les positions 3 et 4 du cycle azabicyclooctane est simple ;
à l'état de base ou de sel d'addition à un acide, ainsi qu'à l'état d'hydrate ou de solvat.

**8.** Composé de formule (I) selon l'une quelconque des revendications 1 à 4, **caractérisé en ce qu'**il est choisi parmi les composés suivants :

- 5-[2-(1-méthyl-1*H*-pyrazol-4-yl)pyridin-5-yl]-1-azabicyclo[3.2.1]-octane ;
- 5-[2-(1*H*-pyrazol-4-yl)pyridin-5-yl]-1-azabicyclo[3.2.1]-octane ;
- 5-[2-(1*H*-pyrazol-4-yl)pyridin-5-yl]-1-azabicyclo[3.2.1]-oct-3-ène ;
- 5-[2-(1-méthyl-1*H*-pyrazol-4-yl)pyridin-5-yl]-1-azabicyclo[3.2.1]-oct-3-ène ;
- 5-[2-(-1*H*-imidazol-1-yl)pyridin-5-yl]-1-azabicyclo[3.2.1]oct-3-ène
- 5-[2-(-1*H*-imidazol-4-yl)pyridin-5-yl]-1-azabicyclo[3.2.]oct-3-ène
- 5-[2-(-1*H*-imidazol-4-yl)pyridin-5-yl]-1-azabicyclo[3.2.1]octane
- 5-[2-(-1*H*-imidazol-1-yl)pyridin-5-yl]-1-azabicyclo[3.2.1]octane
- 5-[2-(-1*H*-imidazol-2-yl)pyridin-5-yl]-1-azabicyclo[3.2.1]octane
- 5-[2-(3,5-diméthyl-1*H*-pyrazol-4-yl)pyridin-5-yl]-1-azabicyclo[3.2.1]-octane ;
- 5-[2-(1*H*-1,2,4-triazol-3-yl)pyridin-5-yl]-1-azabicyclo[3.2.1]-octane ;
- 5-[2-(5-méthyl-1,2,4-oxadiazol-3-yl)pyridin-5-yl]-1-azabicyclo[3.2.1]-octane ;
- 5-[2-(1,3-oxazol-2-yl)pyridin-5-yl]-1-azabicyclo[3.2.1]-octane ;
- 5-[2-(thiazol-4-yl)pyridin-5-yl]-1-azabicyclo[3.2.1]octane
- 5-[2-(pyrazol-3-yl)pyridin-5-yl]-1-azabicyclo[3.2.1]octane
- 5-[2-(2-méthyl-thiazol-5-yl)pyridin-5-yl]-1-azabicyclo[3.2.1]octane
- 5-[2-(tétrazol-5-yl)pyridin-5-yl]-1-azabicyclo[3.2.1]-octane ;
- 5-[2-(1-isobutyl-1*H*-pyrazol-4-yl)pyridin-5-yl]-1-azabicyclo[3.2.1]-octane ;
- 5-[2-(1-*n*-propyl-1*H*-pyrazol-4-yl)pyridin-5-yl]-1-azabicyclo[3.2.1]-octane;

à l'état de base ou de sel d'addition à un acide, ainsi qu'à l'état d'hydrate ou de solvat :
à l'état d'énantiomère pur ou de mélange d'énantiomères.

**9.** Composé de formule (I) selon l'une quelconque des revendications 1 à 4, **caractérisé en ce qu'**il est choisi parmi les composés suivants :

- (-)-Bromhydrate (2 :1) de 5-[2-(1-méthyl-1*H*-pyrazol-4-yl)pyridin-5-yl]-1-azabicyclo[3.2.1]-octane
- Bromhydrate (1:1) de 5-[2-(1*H*-pyrazol-4-yl)pyridin-5-yl]-1-azabicyclo[3.2.1]-octane
- Bromhydrate (2:1) de 5-[2-(1*H*-pyrazol-4-yl)pyridin-5-yl]-1-azabicyclo[3.2.1]-oct-3-ène ;
- Bromhydrate (2:1) de 5-[2-(1-méthyl-1*H*-pyrazol-4-yl)pyridin-5-yl]-1-azabicyclo[3.2.1]-oct-3-ène ;
- Bromhydrate (1:1) de 5-[2-(-1*H*-imidazol-1-yl)pyridin-5-yl]-1-azabicyclo[3.2.1]oct-3-ène
- Chlorhydrate (3:1) de 5-[2-(-1*H*-imidazol-4-yl)pyridin-5-yl]-1-azabicyclo[3.2.1]oct-3-ène
- (-)-Chlorhydrate (3:1) de 5-[2-(-1*H*-imidazol-4-yl)pyridin-5-yl]-1-azabicyclo[3.2.1]octane
- (-)-Bromhydrate (1:1) de 5-[2-(-1*H*-imidazol-1-yl)pyridin-5-yl]-1-azabicyclo[3.2.1]octane
- (+)-Chlorhydrate (2:1) de 5-[2-(-1*H*-imidazol-4-yl)pyridin-5-yl]-1-azabicyclo[3.2.1]octane-
- (-)-Chlorhydrate (2:1) de 5-[2-(-1*H*-imidazol-2-yl)pyridin-5-yl]-1-azabicyclo[3.2.1]octane
- (+)-Bromhydrate (1:1) de 5-[2-(-1*H*-imidazol-1-yl)pyridin-5-yl]-1-azabicyclo[3.2.1]octane
- (+)-Bromhydrate (2:1) de 5-[2-(1*H*-pyrazol-4-yl)pyridin-5-yl]-1-azabicyclo[3.2.1]-octane
- (+)-Bromhydrate (2:1) de 5-[2-(1-méthyl-1*H*-pyrazol-4-yl)pyridin-5-yi]-1-azabicyclo[3.2.1]-octane
- (-)-Bromhydrate (2:1) de 5-[2-(-1*H*-imidazol-2-yl)pyridin-5-yl]-1-azabicyclo[3.2.1]octane
- (+)-Bromhydrate (2:1) de 5-[2-(3,5-diméthyl-1*H*-pyrazol-4-yl)pyridin-5-yl]-1-azabicyclo[3.2.1]-octane
- (-)-Bromhydrate (2 :1) de 5-[2-(1*H*-1,2,4-triazol-3-yl)pyridin-5-yl]-1-azabicyclo[3.2.1]-octane ;
- (-)-Bromhydrate (1:1) de 5-[2-(5-méthyl-1,2,4-oxadiazol-3-yl)pyridin-5-yl]-1-azabicyclo[3.2.1]-octane ;
- (-)-5-[2-(1,3-oxazol-2-yl)pyridin-5-yl]-1-azabicyclo[3.2.1]-octane ;
- (-)-Bromhydrate (1:1) de 5-[2-(thiazol-4-yl)pyridin-5-yl]-1-azabicyclo[3.2.1]octane
- (+)-Bromhydrate (1:1) de 5-[2-(pyrazol-3-yl)pyridin-5-yl]-1-azabicyclo[3.2.1]octane
- (-)-Bromhydrate (1:1) de 5-[2-(2-méthyl-thiazol-5-yl)pyridin-5-yl]-1-azabicyclo[3.2.1]octane
- (+)-Bromhydrate (2 :1) de 5-[2-(1*H*-1,2,4-triazol-3-yl)pyridin-5-yl]-1-azabicyclo[3.2.1]-octane
- (+)-5-[2-(tétrazol-5-yl)pyridin-5-yl]-1-azabicyclo[3.2.1]-octane ;
- (+)-(S,S)-Dibenzoyl tartrate (1 :1) de 5-[2-(1-méthyl-1*H*-pyrazol-4-yl)pyridin-5-yl]-1-aza-bicyclo-[3.2.1]-octane
- (+)-5-[2-(1-méthyl-1*H*-pyrazol-4-yl)pyridin-5-yl]-1-azabicyclo[3.2.1]-octane
- (+)-Fumarate (1 :1) de 5-[2-(1-méthyl-1*H*-pyrazol-4-yl)pyridin-5-yl]-1-azabicyclo[3.2.1]-octane
- (+)-Chlorhydrate (2 :1) de 5-[2-(1-méthyl-1*H*-pyrazol-4-yl)pyridin-5-yl]-1-azabicyclo[3.2.1]-octane
- (-)-Bromhydrate (2:1) de 5-[2-(1-isobutyl-1*H*-pyrazol-4-yl)pyridin-5-yl]-1-azabicyclo[3.2.1]-octane ;
- (-)-Bromhydrate (2:1) de 5-[2-(1-*n*-propyl-1*H*-pyrazol-4-yl)pyridin-5-yl]-1-azabicyclo[3.2.1]-octane.

**10.** Procédé de préparation d'un composé de formule (I) selon l'une quelconque des revendications 1 à 9, **caractérisé en ce que** l'on fait réagir un composé de formule (V) ou (VI),

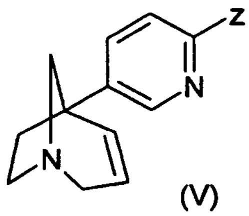
(V)

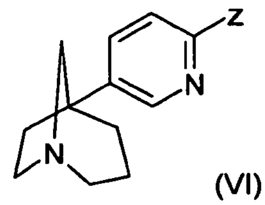
(VI)

dans laquelle Z représente un atome de brome,
soit avec un acide boronique de formule R-$B(OH)_2$ dans laquelle R est tel que défini dans la formule générale (I), en présence d'un catalyseur au palladium ;
soit avec un composé de formule R-H dans laquelle R est tel que défini dans la formule générale (I), en présence d'une base forte dans un solvant ;
soit avec un dérivé stanneux de formule R-$Sn[(CH_2)_3CH_3)]_3$ dans laquelle R est tel que défini dans la formule générale (I), en présence d'un catalyseur au palladium ;
soit avec un composé de formule R-H dans laquelle R est tel que défini dans la formule générale (I), en présence de n-butyllithium, de chlorure de zinc et d'un catalyseur au palladium.

**11.** Procédé de préparation d'un composé de la formule (I)

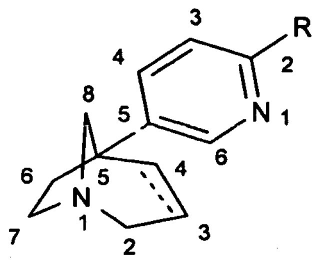
(I)

dans laquelle :

R représente un groupe choisi parmi un triazolyle, oxadiazolyle, tétrazolyle, ce groupe pouvant éventuellement être substitué par un ou plusieurs groupes choisis parmi les atomes d'halogènes, les groupes $(C_1-C_6)$alkyle, $(C_1-C_6)$alcoxy, trifluorométhoxy, trifluorométhyle, nitro, cyano, hydroxy, amino, $(C_1-C_6)$alkylamino ou di$(C_1-C_6)$alkylamino ;
la liaison carbone-carbone entre les positions 3 et 4 du cycle azabicyclooctane est simple,

**caractérisé en ce que**

- on fait réagir un composé de formule (VI)

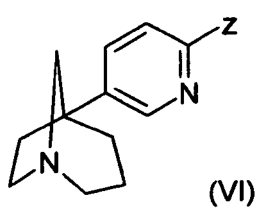
(VI)

dans laquelle Z représente un atome de brome,

en présence de cyanure de potassium et de tétrakis(triphénylphosphino)-palladium dans un solvant, pour obtenir un composé de formule (VII)

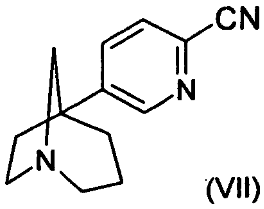

(VII)

puis

- lorsque R représente un groupement triazolyle, on fait réagir le composé de formule (VII) en présence d'une base forte avec de l'hydrazide formique dans un solvant ;
- lorsque R représente un groupement oxadiazolyle, on transforme le composé de formule (VII) en N- hydroxy-carboxamidine de formule (VIII)

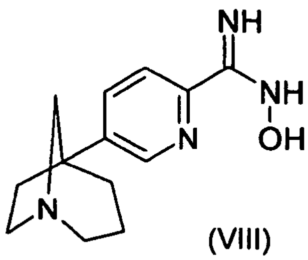

(VIII)

en présence de chlorhydrate d'hydroxylamine en milieu basique,
puis on fait réagir le composé de formule (VIII) avec l'anhydride acétique dans un solvant ;

- lorsque R représente un groupement tétrazolyle, on fait réagir le composé de formule (VII) avec de l'azoture de sodium en présence de chlorure d'ammonium dans un solvant.

**12.** Composé de formule (VII)

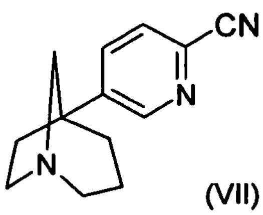

(VII)

**13.** Composé de formule (VIII)

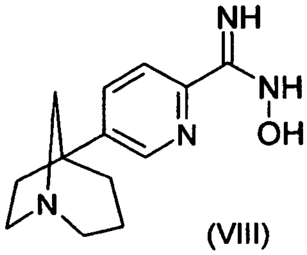

(VIII)

**14.** Médicament, **caractérisé en ce qu'**il comprend un composé de formule (I) selon l'une quelconque des revendications 1 à 9, ou un sel d'addition de ce composé à un acide pharmaceutiquement acceptable, ou encore un hydrate ou un solvat.

**15.** Composition pharmaceutique, **caractérisée en ce qu'**elle comprend un composé de formule (I) selon l'une quelconque des revendications 1 à 9, ou un sel pharmaceutiquement acceptable, un hydrate ou un solvat de ce composé, ainsi qu'au moins un excipient pharmaceutiquement acceptable.

**16.** Utilisation d'un composé de formule (I) selon l'une quelconque des revendications 1 à 9 pour la préparation d'un médicament destiné au traitement et à la prévention des altérations cognitives ; des atteintes des processus attentionnels ; des troubles des fonctions exécutives liées à la maladie d'Alzheimer, au vieillissement pathologique ou normal, au syndrome Parkinsonien, à la trisomie 21, aux pathologies psychiatriques, au syndrome alcoolique de Korsakoff, aux démences vasculaires, aux traumatismes crâniens ; des troubles moteurs observés dans la maladie de Parkinson ou d'autres maladies neurologiques ou des atteintes anatomo-histo-pathologiques liées aux maladies neuro-dégénératives susnommées.

**17.** Utilisation d'un composé de formule (I) selon l'une quelconque des revendications 1 à 9 pour la préparation d'un médicament destiné au traitement et à la prévention des accidents vasculaires cérébraux, des épisodes hypoxiques cérébraux, des pathologies psychiatriques.

**18.** Utilisation d'un composé de formule (I) selon l'une quelconque des revendications 1 à 9 pour la préparation d'un médicament destiné à la prévention des symptômes dus au sevrage au tabac, à l'alcool, aux différentes substances induisant une dépendance.

**19.** Utilisation d'un composé de formule (I) selon l'une quelconque des revendications 1 à 9 pour la préparation d'un médicament destiné au traitement de la douleur.

**20.** Utilisation d'un composé de formule (I) selon l'une quelconque des revendications 1 à 9 pour la préparation d'un médicament destiné au traitement de l'ischémie des membres inférieurs, de l'artérite oblitérante des membres inférieurs, de l'ischémie cardiaque, de l'infarctus du myocarde, de l'insuffisance cardiaque, du déficit de cicatrisation cutanée des patients diabétiques, des ulcères variqueux de l'insuffisance veineuse.

**21.** Utilisation d'un composé de formule (I) selon l'une quelconque des revendications 1 à 9 pour la préparation d'un médicament destiné au traitement des processus inflammatoires.

## Claims

**1.** Compound corresponding to formula (I)

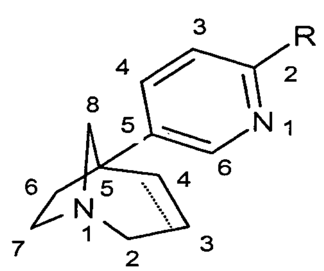

(I)

in which:

R represents a group chosen from pyrazolyl, imidazolyl, triazolyl, oxazolyl, oxadiazolyl, thiazolyl, isothiazolyl, thiadiazolyl and tetrazolyl, this group possibly being substituted with one or more groups chosen from halogen atoms and $(C_1-C_6)$ alkyl, $(C_1-C_6)$ alkoxy, trifluoromethoxy, trifluoromethyl, nitro, cyano, hydroxyl, amino, $(C_1-C_6)$ alkylamino and di$(C_1-C_6)$alkylamino groups;

the carbon-carbon bond between positions 3 and 4 of the azabicyclooctane ring is a single or double bond; in the form of base or of acid-addition salt, and also in the form of hydrate or solvate.

**2.** Compound of formula (I) according to Claim 1, **characterized in that** R represents a group chosen from pyrazolyl, imidazolyl, triazolyl, oxazolyl, oxadiazolyl, thiazolyl, isothiazolyl, thiadiazolyl and tetrazolyl, this group possibly being substituted with one or more ($C_1$-$C_6$)alkyl groups; and the carbon-carbon bond between positions 3 and 4 of the azabicyclooctane ring is a single or double bond; in the form of base or of acid-addition salt, and also in the form of hydrate or solvate.

**3.** Compound of formula (I) according to Claim 1, **characterized in that** R represents a group chosen from pyrazolyl, imidazolyl, triazolyl, oxazolyl, oxadiazolyl, thiazolyl and tetrazolyl, this group possibly being substituted with one or more groups chosen from halogen atoms and ($C_1$-$C_6$) alkyl, ($C_1$-$C_6$) alkoxy, trifluoromethoxy, trifluoromethyl, nitro, cyano, hydroxyl, amino, ($C_1$-$C_6$) alkylamino and di ($C_1$-$C_6$) alkylamino groups; and the carbon-carbon bond between positions 3 and 4 of the azabicyclooctane ring is a single or double bond; in the form of base or of acid-addition salt, and also in the form of hydrate or solvate.

**4.** Compound of formula (I) according to Claim 1 or 3, **characterized in that** R represents a group chosen from pyrazolyl, imidazolyl, triazolyl, oxazolyl, oxadiazolyl, thiazolyl and tetrazolyl, this group possibly being substituted with one or more ($C_1$-$C_6$) alkyl groups; and the carbon-carbon bond between positions 3 and 4 of the azabicyclooctane ring is a single or double bond; in the form of base or of acid-addition salt, and also in the form of hydrate or solvate.

**5.** Compound of formula (I) according to Claim 1 or 2, **characterized in that** R represents a pyrazolyl group optionally substituted with one or more ($C_1$-$C_6$) alkyl, ($C_1$-$C_6$) alkoxy, trifluoromethoxy, trifluoromethyl, nitro, cyano, hydroxyl, amino, ($C_1$-$C_6$) alkylamino or di ($C_1$-$C_6$) alkylamino groups; the carbon-carbon bond between positions 3 and 4 of the azabicyclooctane ring is a single or double bond; in the form of base or of acid-addition salt, and also in the form of hydrate or solvate.

**6.** Compound of formula (I) according to any one of Claims 1 to 5, **characterized in that** R represents a pyrazolyl group optionally substituted with one or more ($C_1$-$C_6$) alkyl groups; the carbon-carbon bond between positions 3 and 4 of the azabicyclooctane ring is a single or double bond; in the form of base or of acid-addition salt, and also in the form of hydrate or solvate.

**7.** Compound of formula (I) according to any one of Claims 1 to 6, **characterized in that** R represents a pyrazolyl group optionally substituted with one or more ($C_1$-$C_6$) alkyl groups; the carbon-carbon bond between positions 3 and 4 of the azabicyclooctane ring is a single bond; in the form of base or of acid-addition salt, and also in the form of hydrate or solvate.

**8.** Compound of formula (I) according to any one of Claims 1 to 4, **characterized in that** it is chosen from the following compounds:

- 5-[2-(1-methyl-1*H*-pyrazol-4-yl)pyrid-5-yl]-1-azabicyclo[3.2.1]octane;
- 5-[2-(1*H*-pyrazol-4-yl)pyrid-5-yl]-1-azabicyclo[3.2.1]octane;
- 5-[2-(1*H*-pyrazol-4-yl)pyrid-5-yl]-1-azabicyclo[3.2.1]oct-3-ene;
- 5-[2-(1-methyl-1*H*-pyrazol-4-yl)pyrid-5-yl]-1-azabicyclo[3.2.1]-oct-3-ene;
- 5-[2-(1*H*-imidazol-1-yl)pyrid-5-yl]-1-azabicyclo[3.2.1]oct-3-ene
- 5-[2-(1*H*-imidazol-4-yl)pyrid-5-yl]-1-azabicyclo[3.2.1]oct-3-ene
- 5-[2-(1*H*-imidazol-4-yl)pyrid-5-yl]-1-azabicyclo[3.2.1]octane
- 5-[2-(1*H*-imidazol-1-yl)pyrid-5-yl]-1-azabicyclo[3.2.1]octane
- 5-[2-(1*H*-imidazol-2-yl)pyrid-5-yl]-1-azabicyclo[3.2.1]octane
- 5-[2-(3,5-dimethyl-1*H*-pyrazol-4-yl)pyrid-5-yl]-1-azabicyclo[3.2.1]octane;
- 5-[2-(1*H*-1,2,4-triazol-3-yl)pyrid-5-yl]-1-azabicyclo[3.2.1]octane;
-5-[2-(5-methyl-1,2,4-oxadiazol-3-yl)pyrid-5-yl]-1-azabicyclo[3.2.1]octane;
- 5-[2-(1,3-oxazol-2-yl)pyrid-5-yl]-1-azabicyclo[3.2.1]octane;
- 5-[2-(thiazol-4-yl)pyrid-5-yl]-1-azabicyclo[3.2.1]octane
- 5-[2-(pyrazol-3-yl)pyrid-5-yl]-1-azabicyclo[3.2.1]octane
- 5-[2-(2-methylthiazol-5-yl)pyrid-5-yl]-1-azabicyclo[3.2.1]octane

- 5-[2-(tetrazol-5-yl)pyrid-5-yl]-1-azabicyclo[3.2.1]octane;
- 5-[2-(1-isobutyl-1*H*-pyrazol-4-yl)pyrid-5-yl]-1-azabicyclo[3.2.1]octane;
- 5-[2-(1-*n*-propyl-1*H*-pyrazol-4-yl)pyrid-5-yl]-1-azabicyclo[3,2,1]octane ;

in the form of base or of acid-addition salt, and also in the form of hydrate or solvate; in the form of a pure enantiomer or a mixture of enantiomers.

**9.** Compound of formula (I) according to any one of Claims 1 to 4, **characterized in that** it is chosen from the following compounds:

- (-)-5-[2-(1-methyl-1*H*-pyrazol-4-yl)pyrid-5-yl]-1-azabicyclo[3.2.1]octane hydrobromide (1:2)
- 5-[2-(1*H*-pyrazol-4-yl)pyrid-5-yl]-1-azabicyclo-[3.2.1]octane hydrobromide (1:1)
- 5-[2-(1*H*-pyrazol-4-yl)pyrid-5-yl]-1-azabicyclo-[3.2.1]oct-3-ene hydrobromide (1:2)
- 5-[2-(1-methyl-1*H*-pyrazol-4-yl)pyrid-5-yl]-1-azabicyclo[3.2.1]oct-3-ene hydrobromide (1:2)
- 5-[2-(1*H*-imidazol-1-yl)pyrid-5-yl]-1-azabicyclo-[3.2.1]oct-3-ene hydrobromide (1:1)
- 5-[2-(1*H*-imidazol-4-yl)pyrid-5-yl]-1-azabicyclo-[3.2.1]oct-3-ene hydrochloride (1:3)
- (-)-5-[2-(1*H*-imidazol-4-yl)pyrid-5-yl]-1-azabicyclo-[3.2.1]octane hydrochloride (1:3)
- (-)-5-[2-(1*H*-imidazol-1-yl)pyrid-5-yl]-1-azabicyclo-[3.2.1]octane hydrobromide (1:1)
- (+)-5-[2-(1*H*-imidazol-4-yl)pyrid-5-yl]-1-azabicyclo-[3.2.1]octane hydrochloride (1:2)
- (-)-5-[2-(1*H*-imidazol-2-yl)pyrid-5-yl]-1-azabicyclo-[3.2.1]octane hydrochloride (1:2)
- (+)-5-[2-(1*H*-imidazol-1-yl)pyrid-5-yl]-1-azabicyclo-[3.2.1]octane hydrobromide (1:1)
- (+)-5-[2-(1*H*-pyrazol-4-yl)pyrid-5-yl]-1-azabicyclo-[3.2.1]octane hydrobromide (1:2)
- (+)-5-[2-(1-methyl-1*H*-pyrazol-4-yl)pyrid-5-yl]-1-azabicyclo[3.2.1]octane hydrobromide (1:2)
- (-)-5-[2-(1*H*-imidazol-2-yl)pyrid-5-yl]-1-azabicyclo-[3.2.1]octane hydrobromide (1:2)
- (+)-5-[2-(3,5-dimethyl-1*H*-pyrazol-4-yl)pyrid-5-yl]-1-azabicyclo[3.2.1]octane hydrobromide (1:2)
- (-)-5-[2-(1*H*-1,2,4-triazol-3-yl)pyrid-5-yl]-1-azabicyclo[3.2.1]octane hydrobromide (1:2)
- (-)-5-[2-(5-methyl-1,2,4-oxadiazol-3-yl)pyrid-5-yl]-1-azabicyclo[3.2.1]octane hydrobromide (1:1)
- (-)-5-[2-(1,3-oxazol-2-yl)pyrid-5-yl]-1-azabicyclo-[3.2.1]octane
- (-)-5-[2-(thiazol-4-yl)pyrid-5-yl]-1-azabicyclo-[3.2.1]octane hydrobromide (1:1)
- (+)-5-[2-(pyrazol-3-yl)pyrid-5-yl]-1-azabicyclo-[3.2.1]octane hydrobromide (1:1)
- (-)-5-[2-(2-methylthiazol-5-yl)pyrid-5-yl]-1-azabicyclo[3.2.1]octane hydrobromide (1:1)
- (+)-5-[2-(1*H*-1,2,4-triazol-3-yl)pyrid-5-yl]-1-azabicyclo[.3.2.1]octane hydrobromide (1:2)
- (+)-5-[2-(tetrazol-5-yl)pyrid-5-yl]-1-azabicyclo-[3.2.1]octane
- (+)-5-[2-(1-methyl-1*H*-pyrazol-4-yl)pyrid-5-yl]-1-azabicyclo[3.2.1]octane dibenzoyl (S,S)-tartrate (1:1)
- (+)-5-[2-(1-methyl-1*H*-pyrazol-4-yl)pyrid-5-yl]-1-azabicyclo[3.2.1]octane
- (+)-5-[2-(1-methyl-1*H*-pyrazol-4-yl)pyrid-5-yl]-1-azabicyclo[3.2.1]octane fumarate (1:1)
- (+)-5-[2-(1-methyl-1*H*-pyrazol-4-yl)pyrid-5-yl]-1-azabicyclo[3.2.1]octane hydrochloride (1:2)
- (-)-5-[2-(1-isobutyl-1*H*-pyrazol-4-yl)pyrid-5-yl]-1-azabicyclo[3.2.1]octane hydrobromide (1:2)
- (-)-5-[2-(1-*n*-propyl-1*H*-pyrazol-4-yl)pyrid-5-yl]-1-azabicyclo[3.2.1]octane hydrobromide (1:2).

**10.** Process for preparing a compound of formula (I) according to any one of Claims 1 to 9, **characterized in that** a compound of formula (V) or (VI),

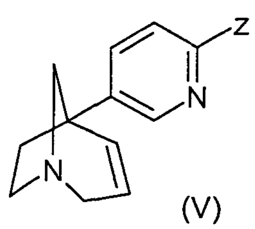
(V)

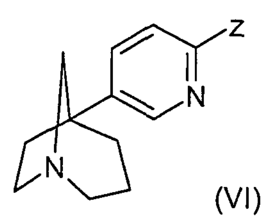
(VI)

in which Z represents a bromine atom,
is reacted either with a boronic acid of formula R-$B(OH)_2$ in which R is as defined in the general formula (I), in the presence of a palladium catalyst;
or with a compound of formula R-H in which R is as defined in the general formula (I), in the presence of a strong base in a solvent;
or with a stannous derivative of formula R-$Sn[(CH_2)_3CH_3)]_3$ in which R is as defined in the general formula (I), in the presence of a palladium catalyst; or with a compound of formula R-H in which R is as defined in the general

formula (I), in the presence of n-butyllithium, zinc chloride and a palladium catalyst.

**11.** Process for preparing a compound of formula (I)

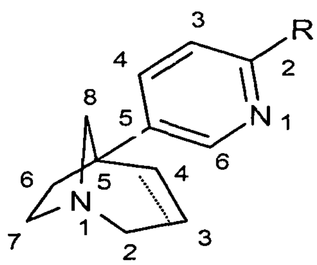

(I)

in which:

R represents a group chosen from triazolyl, oxadiazolyl and tetrazolyl, this group possibly being substituted with one or more groups chosen from halogen atoms and ($C_1$-$C_6$) alkyl, ($C_1$-$C_6$) alkoxy, trifluoromethoxy, trifluoromethyl, nitro, cyano, hydroxyl, amino, ($C_1$-$C_6$) alkylamino and di ($C_1$-$C_6$) alkylamino groups;
the carbon-carbon bond between positions 3 and 4 of the azabicyclooctane ring is a single bond,

**characterized in that**

- a compound of formula (VI)

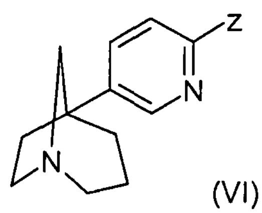

(VI)

in which Z represents a bromine atom,
is reacted in the presence of potassium cyanide and tetrakis(triphenylphosphine)palladium in a solvent,
to obtain a compound of formula (VII)

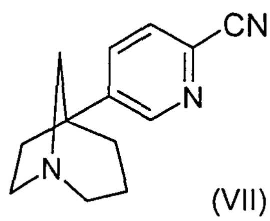

(VII)

and then

- when R represents a triazolyl group, the compound of formula (VII) is reacted in the presence of a strong base with formic hydrazide in a solvent;
- when R represents an oxadiazolyl group, the compound of formula (VII) is converted into the N- hydroxycarboxamidine of formula (VIII)

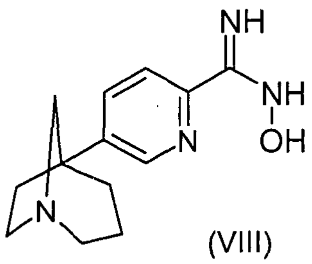

(VIII)

in the presence of hydroxylamine hydrochloride in basic medium,
and the compound of formula (VIII) is then reacted with acetic anhydride in a solvent;

- when R represents a tetrazolyl group, the compound of formula (VII) is reacted with sodium azide in the presence of ammonium chloride in a solvent.

**12.** Compound of formula (VII)

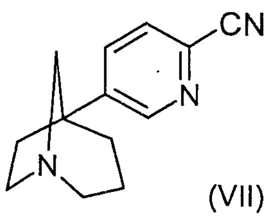

(VII)

**13.** Compound of formula (VIII)

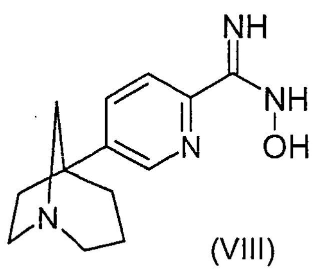

(VIII)

**14.** Medicament, **characterized in that** it comprises a compound of formula (I)-according to any one of Claims 1 to 9, or an addition salt of this compound with a pharmaceutically acceptable acid, or a hydrate or a solvate.

**15.** Pharmaceutical composition, **characterized in that** it comprises a compound of formula (I) according to any one of Claims 1 to 9, or a pharmaceutically acceptable salt, or a hydrate or solvate of this compound, and also at least one pharmaceutically acceptable excipient.

**16.** Use of a compound of formula (I) according to any one of Claims 1 to 9 for the preparation of a medicament for treating and preventing cognitive impairment; attention impairment; executive function disorders associated with Alzheimer's disease, pathological or normal ageing, Parkinson's disease, trisomy 21, psychiatric pathologies, Korsakoff's alcoholic syndrome, vascular dementia, or cranial trauma; motor disorders observed in Parkinson's disease or other neurological diseases or anatomo-histopathological complaints associated with the abovementioned neurodegenerative diseases.

**17.** Use of a compound of formula (I) according to any one of Claims 1 to 9 for the preparation of a medicament for treating and preventing strokes, cerebral hypoxic episodes and psychiatric pathologies.

**18.** Use of a compound of formula (I) according to any one of Claims 1 to 9 for the preparation of a medicament for preventing symptoms caused by weaning from tobacco, from alcohol or from various substances that induce dependency.

**19.** Use of a compound of formula (I) according to any one of Claims 1 to 9 for the preparation of a medicament for treating pain.

**20.** Use of a compound of formula (I) according to any one of Claims 1 to 9 for the preparation of a medicament for treating ischaemia of the lower limbs, obliterative arteritis of the lower limbs, cardiac ischaemia, myocardial infarction, cardiac insufficiency, skin cicatrization deficiency of diabetic patients, and varicose ulcers of venous insufficiency.

**21.** Use of a compound of formula (I) according to any one of Claims 1 to 9 for the preparation of a medicament for treating inflammatory processes.

**Patentansprüche**

**1.** Verbindung der Formel (I)

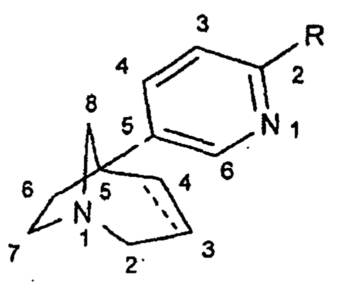

(I)

worin:

R für eine unter Pyrazolyl, Imidazolyl, Triazolyl, Oxazolyl, Oxadiazolyl, Thiazolyl, Isothiazolyl, Thiadiazolyl und Tetrazolyl ausgewählte Gruppe steht, wobei diese Gruppe gegebenenfalls durch eine oder mehrere unter Halogenatomen und ($C_1$-$C_6$) - Alkyl-, ($C_1$-$C_6$)-Alkoxy-, Trifluormethoxy-, Trifluormethyl-, Nitro-, Cyano-, Hydroxy-, Amino-, ($C_1$-$C_6$) -Alkylamino- und Di- ($C_1$-$C_6$) -alkylaminogruppen ausgewählte Gruppen substituiert sein kann; und
die Kohlenstoff-Kohlenstoff-Bindung zwischen den Positionen 3 und 4 des Azabicyclooctans eine Einfachbindung oder eine Doppelbindung ist;
in Basen- oder Säureadditionssalzform sowie in Hydrat- oder Solvatform.

**2.** Verbindung der Formel (I) nach Anspruch 1, **dadurch gekennzeichnet, daß**
R für eine unter Pyrazolyl, Imidazolyl, Triazolyl, Oxazolyl, Oxadiazolyl, Thiazolyl, Isothiazolyl, Thiadiazolyl und Tetrazolyl ausgewählte Gruppe steht, wobei diese Gruppe gegebenenfalls durch eine oder mehrere ($C_1$-$C_6$)-Alkylgruppen substituiert sein kann; und
die Kohlenstoff-Kohlenstoff-Bindung zwischen den Positionen 3 und 4 des Azabicyclooctans eine Einfachbindung oder eine Doppelbindung ist;
in Basen- oder Säureadditionssalzform sowie in Hydrat- oder Solvatform.

**3.** Verbindung der Formel (I) nach Anspruch 1, **dadurch gekennzeichnet, daß**
R für eine unter Pyrazolyl, Imidazolyl, Triazolyl, Oxazolyl, Oxadiazolyl, Thiazolyl und Tetrazolyl ausgewählte Gruppe steht, wobei diese Gruppe gegebenenfalls durch eine oder mehrere unter Halogenatomen und ($C_1$-$C_6$) -Alkyl-, ($C_1$-$C_6$) -Alkoxy-, Trifluormethoxy-, Trifluormethyl-, Nitro-, Cyano-, Hydroxy-, Amino-, ($C_1$-$C_6$)-Alkylamino- und Di-($C_1$-$C_6$)-alkylaminogruppen ausgewählte Gruppen substituiert sein kann; und
die Kohlenstoff-Kohlenstoff-Bindung zwischen den Positionen 3 und 4 des Azabicyclooctans eine Einfachbindung oder eine Doppelbindung ist;
in Basen- oder Säureadditionssalzform sowie in Hydrat- oder Solvatform.

4. Verbindung der Formel (I) nach Anspruch 1 oder 3, **dadurch gekennzeichnet, daß** R für eine unter Pyrazolyl, Imidazolyl, Triazolyl, Oxazolyl, Oxadiazolyl, Thiazolyl und Tetrazolyl ausgewählte Gruppe steht, wobei diese Gruppe gegebenenfalls durch eine oder mehrere ($C_1$-$C_6$) - Alkylgruppen substituiert sein kann; und die Kohlenstoff-Kohlenstoff-Bindung zwischen den Positionen 3 und 4 des Azabicyclooctans eine Einfachbindung oder eine Doppelbindung ist;
in Basen- oder Säureadditionssalzform sowie in Hydrat- oder Solvatform.

5. Verbindung der Formel (I) nach Anspruch 1 oder 2, **dadurch gekennzeichnet, daß** R für eine Pyrazolylgruppe steht, die gegebenenfalls durch eine oder mehrere ($C_1$-$C_6$) -Alkyl-, ($C_1$-$C_6$)-Alkoxy-, Trifluormethoxy-, Trifluormethyl-, Nitro-, Cyano-, Hydroxy-, Amino-, ($C_1$-$C_6$)-Alkylamino- oder Di-($C_1$-$C_6$)-alkylaminogruppen substituiert ist; und
die Kohlenstoff-Kohlenstoff-Bindung zwischen den Positionen 3 und 4 des Azabicyclooctans eine Einfachbindung oder eine Doppelbindung ist;
in Basen- oder Säureadditionssalzform sowie in Hydrat- oder Solvatform.

6. Verbindung der Formel (I) nach einem der Ansprüche 1 bis 5, **dadurch gekennzeichnet, daß** R für eine Pyrazolylgruppe steht, die gegebenenfalls durch eine oder mehrere ($C_1$-$C_6$)-Alkylgruppen substituiert ist; und
die Kohlenstoff-Kohlenstoff-Bindung zwischen den Positionen 3 und 4 des Azabicyclooctans eine Einfachbindung oder eine Doppelbindung ist;
in Basen- oder Säureadditionssalzform sowie in Hydrat- oder Solvatform.

7. Verbindung der Formel (I) nach einem der Ansprüche 1 bis 6, **dadurch gekennzeichnet, daß** R für eine Pyrazolylgruppe steht, die gegebenenfalls durch eine oder mehrere ($C_1$-$C_6$)-Alkylgruppen substituiert ist; und
die Kohlenstoff-Kohlenstoff-Bindung zwischen den Positionen 3 und 4 des Azabicyclooctans eine Einfachbindung ist;
in Basen- oder Säureadditionssalzform sowie in Hydrat- oder Solvatform.

8. Verbindung der Formel (I) nach einem der Ansprüche 1 bis 4, **dadurch gekennzeichnet, daß** sie unter den folgenden Verbindungen ausgewählt ist:

- 5-[2-(1-Methyl-1*H*-pyrazol-4-yl)pyridin-5-yl]-1-azabicyclo[3.2.1]octan;
- 5-[2-(1*H*-Pyrazol-4-yl)pyridin-5-yl]-1-azabicyclo[3.2.1]octan;
- 5-[2-(1*H*-Pyrazol-4-yl)pyridin-5-yl]-1-azabicyclo[3.2.1]oct-3-en;
- 5-[2-(1-Methyl-1*H*-pyrazol-4-yl)pyridin-5-yl]-1-azabicyclo[3.2.1]oct-3-en;
- 5-[2-(1*H*-Imidazol-1-yl)pyridin-5-yl]-1-azabicyclo[3.2.1]oct-3-en;
- 5-[2-(1*H*-Imidazol-4-yl)pyridin-5-yl]-1-azabicyclo[3.2.1]oct-3-en;
- 5-[2-(1*H*-Imidazol-4-yl)pyridin-5-yl]-1-azabicyclo[3.2.1]octan;
- 5-[2-(1*H*-Imidazol-1-yl)pyridin-5-yl]-1-azabicyclo[3.2.1]octan;
- 5-[2-(1*H*-Imidazol-2-yl)pyridin-5-yl]-1-azabicyclo[3.2.1]octan;
- 5-[2-(3,5-Dimethyl-1*H*-pyrazol-4-yl)pyridin-5-yl]-1-azabicyclo[3.2.1]octan;
- 5-[2-(1*H*-1,2,4-Triazol-3-yl)pyridin-5-yl]-1-azabicyclo[3.2.1]octan;
- 5-[2-(5-Methyl-1,2,4-oxadiazol-3-yl)pyridin-5-yl]-1-azabicyclo[3.2.1]octan;
- 5-[2-(1,3-Oxazol-2-yl)pyridin-5-yl]-1-azabicyclo[3.2.1]octan;
- 5-[2-(Thiazol-4-yl)pyridin-5-yl]-1-azabicyclo-[3.2.1]octan;
- 5-[2-(Pyrazol-3-yl)pyridin-5-yl]-1-azabicyclo-[3.2.1]octan;
- 5-[2-(2-Methylthiazol-5-yl)pyridin-5-yl]-1-azabicyclo[3.2.1]octan;
- 5-[2-(Tetrazol-5-yl)pyridin-5-yl]-1-azabicyclo-[3.2.1]octan;
- 5-[2-(1-Isobutyl-1*H*-pyrazol-4-yl)pyridin-5-yl]-1-azabicyclo[3.2.1]octan;
- 5-[2-(1-*n*-Propyl-1*H*-pyrazol-4-yl)pyridin-5-yl]-1-azabicyclo[3.2.1]octan;

in Basen- oder Säureadditionssalzform sowie in Hydrat- oder Solvatform;
in Form eines reinen Enantiomers oder eines Enantiomerengemischs.

9. Verbindung der Formel (I) nach einem der Ansprüche 1 bis 4, **dadurch gekennzeichnet, daß** sie unter den folgenden Verbindungen ausgewählt ist:

- (-)5-[2-(1-Methyl-1*H*-pyrazol-4-yl)pyridin-5-yl]-1-azabicyclo[3.2.1]octan-hydrobromid (1:2);
- 5-[2-(1*H*-Pyrazol-4-yl)pyridin-5-yl]-1-azabicyclo[3.2.1]octan-hydrobromid (1:1);

- 5-[2-(1*H*-Pyrazol-4-yl)pyridin-5-yl]-1-azabicyclo[3.2.1]oct-3-en-hydrobromid (1:2);
- 5-[2-(1-Methyl-1*H*-pyrazol-4-yl)pyridin-5-yl]-1-azabicyclo[3.2.1]oct-3-en-hydrobromid (1:2);
- 5-[2-(1*H*-Imidazol-1-yl)pyridin-5-yl]-1-azabicyclo[3.2.1]oct-3-en-hydrobromid (1:1);
- 5-[2-(1*H*-Imidazol-4-yl)pyridin-5-yl]-1-azabicyclo[3.2.1]oct-3-en-hydrochlorid (1:3);
- (-)-5-[2-(1*H*-Imidazol-4-yl)pyridin-5-yl]-1-azabicyclo[3.2.1]octan-hydrochlorid (1:3);
- (-)-5-[2-(1*H*-Imidazol-1-yl)pyridin-5-yl]-1-azabicyclo[3.2.1]octan-hydrobromid (1:1);
- (+)-5-[2-(1*H*-Imidazol-2-yl)pyridin-5-yl]-1-azabicyclo[3.2.1]octan-hydrochlorid (1:2);
- (-)-5-[2-(1*H*-1midazol-2-yl)pyridin-5-yl]-1-azabicyclo[3.2.1]octan-hydrochlorid (1:2);
- (+)-5-[2-(1*H*-Imidazol-1-yl)pyridin-5-yl]-1-azabicyclo[3.2.1]octan-hydrobromid (1:1);
- (+)-5-[2-(1*H*-Pyrazol-4-yl)pyridin-5-yl]-1-azabicyclo[3.2.1]octan-hydrobromid (1:2);
- (+)-5-[2-(1-Methyl-1*H*-pyrazol-4-yl)pyridin-5-yl]-1-azabicyclo[3.2.1]octan-hydrobromid (1:2);
- (-)-5-[2-(1*H*-Imidazol-2-yl)pyridin-5-yl]-1-azabicyclo[3.2.1]octan-hydrobromid (1:2);
- (+)-5-[2-(3,5-Dimethyl-1*H*-pyrazol-4-yl)pyridin-5-yl]-1-azabicyclo[3.2.1]octan-hydrobromid (1:2);
- (-)-5-[2-(1*H*-1,2,4-Triazol-3-yl)pyridin-5-yl]-1-azabicyclo[3.2.1]octan-hydrobromid (1:2);
- (-)-5-[2-(5-Methyl-1,2,4-oxadiazol-3-yl)pyridin-5-yl]-1-azabicyclo[3.2.1]octan-hydrobromid (1:1);
- (-)-5-[2-(1,3-Oxazol-2-yl)pyridin-5-yl]-1-azabicyclo[3.2.1]octan;
- (-)-5-[2-(Thiazol-4-yl)pyridin-5-yl]-1-azabicyclo[3.2.1]octan-hydrobromid (1:1);
- (+)-5-[2-(Pyrazol-3-yl)pyridin-5-yl]-1-azabicyclo[3.2.1]octan-hydrobromid (1:1);
- (-)-5-[2-(2-Methylthiazol-5-yl)pyridin-5-yl]-1-azabicyclo[3.2.1]octan-hydrobromid (1:1);
- (+)-5-[2-(1*H*-1,2,4-Triazol-3-yl)pyridin-5-yl]-1-azabicyclo[3.2.1]octan-hydrobromid (1:2);
- (+)-5-[2-(Tetrazol-5-yl)pyridin-5-yl]-1-azabicyclo[3.2.1]octan;
- (+)-5-[2-(1-Methyl-1*H*-pyrazol-4-yl)pyridin-5-yl]-1-azabicyclo[3.2.1]octan-(S,S)-dibenzoyltartrat (1:1);
- (+)-5-[2-(1-Methyl-1*H*-pyrazol-4-yl)pyridin-5-yl]-1-azabicyclo[3.2.1]octan;
- (+)-5-[2-(1-Methyl-1*H*-pyrazol-4-yl)pyridin-5-yl]-1-azabicyclo[3.2.1]octan-fumarat (1:1);
- (+)-5-[2-(1-Methyl-1*H*-pyrazol-4-yl)pyridin-5-yl]-1-azabicyclo[3.2.1]octan-hydrochlorid (1:2);
- (-)-5-[2-(1-Isobutyl-1*H*-pyrazol-4-yl)pyridin-5-yl]-1-azabicyclo[3.2.1]octan-hydrobromid (1:2);
- (-)-5-[2-(1-*n*-Propyl-1*H*-pyrazol-4-yl)pyridin-5-yl]-1-azabicyclo[3.2.1]octan-hydrobromid (1:2).

**10.** Verfahren zur Herstellung einer Verbindung der Formel (I) nach einem der Ansprüche 1 bis 9, **dadurch gekennzeichnet, daß** man eine Verbindung der Formel (V) oder (VI)

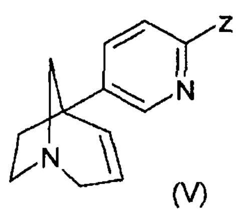
(V)

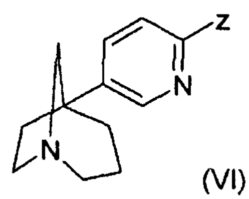
(VI)

worin Z für ein Bromatom steht,
entweder in Gegenwart eines Palladiumkatalysators mit einer Boronsäure der Formel $R\text{-}B(OH)_2$, worin R die in der allgemeinen Formel (I) angegebene Bedeutung besitzt;
oder in Gegenwart einer starken Base in einem Lösungsmittel mit einer Verbindung der Formel R-H, worin R die in der allgemeinen Formel (I) angegebene Bedeutung besitzt;
oder in Gegenwart eines Palladiumkatalysators mit einem Zinn (IV) -Derivat der Formel $R\text{-}Sn[(CH_2)_3CH_3)]_3$, worin R die in der allgemeinen Formel (I) angegebene Bedeutung besitzt;
oder in Gegenwart von n-Butyllithium, Zinkchlorid und einem Palladiumkatalysator mit einer Verbindung der Formel R-H, worin R die in der allgemeinen Formel (I) angegebene Bedeutung besitzt;
umsetzt.

**11.** Verfahren zur Herstellung einer Verbindung der Formel (I)

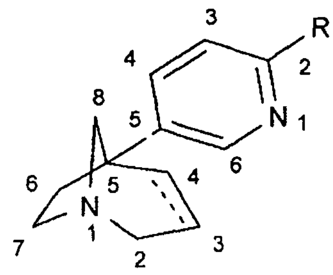

(I)

worin:

R für eine unter Triazolyl, Oxadiazolyl und Tetrazolyl ausgewählte Gruppe steht, wobei diese Gruppe gegebenenfalls durch eine oder mehrere unter Halogenatomen und ($C_1$-$C_6$) -Alkyl-, ($C_1$-$C_6$) - Alkoxy-, Trifluormethoxy-, Trifluormethyl-, Nitro-, Cyano-, Hydroxy-, Amino-, ($C_1$-$C_6$)-Alkylamino- und Di-($C_1$-$C_6$)-alkylaminogruppen ausgewählte Gruppen substituiert sein kann; und
die Kohlenstoff-Kohlenstoff-Bindung zwischen den Positionen 3 und 4 des Azabicyclooctans eine Einfachbindung ist;

**dadurch gekennzeichnet, daß** man

- eine Verbindung der Formel (VI)

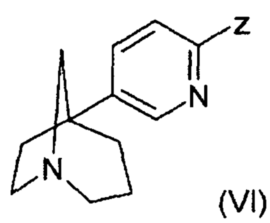

(VI)

worin Z für ein Bromatom steht,
in Gegenwart von Kaliumcyanid und Tetrakis-(triphenylphosphin)palladium in einem Lösungsmittel zu einer Verbindung der Formel (VII)

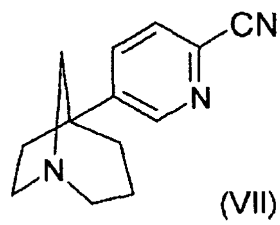

(VII)

umsetzt und dann

- in dem Fall, daß R für eine Triazolylgruppe steht, die Verbindung der Formel (VII) in Gegenwart einer starken Base in einem Lösungsmittel mit Ameisensäurehydrazid umsetzt,
- in dem Fall, daß R für eine Oxadiazolylgruppe steht, die Verbindung der Formel (VII) in Gegenwart von Hydroxylaminhydrochlorid in basischem Milieu zum N-Hydroxycarboxamidin der Formel (VIII)

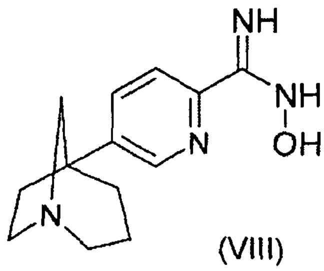

(VIII)

umwandelt und dann die Verbindung der Formel (VIII) in einem Lösungsmittel mit Essigsäureanhydrid umsetzt;
- in dem Fall, daß R für eine Tetrazolylgruppe steht, die Verbindung der Formel (VII) in Gegenwart von Ammoniumchlorid in einem Lösungsmittel mit Natriumazid umsetzt.

**12.** Verbindung der Formel (VII)

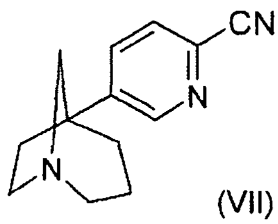

(VII)

**13.** Verbindung der Formel (VIII)

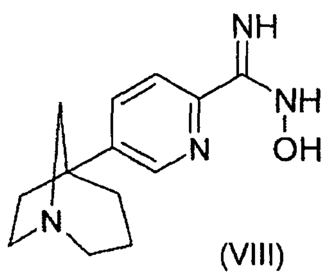

(VIII)

**14.** Arzneimittel, **dadurch gekennzeichnet, daß** es eine Verbindung der Formel (I) nach einem der Ansprüche 1 bis 9 oder ein Additionssalz dieser Verbindung mit einer pharmazeutisch unbedenklichen Säure oder auch ein Hydrat oder ein Solvat enthält.

**15.** Pharmazeutische Zusammensetzung, **dadurch gekennzeichnet, daß** sie eine Verbindung der Formel (I) nach einem der Ansprüche 1 bis 9 oder ein pharmazeutisch unbedenkliches Salz, ein Hydrat oder ein Solvat dieser Verbindung sowie mindestens einen pharmazeutisch unbedenklichen Hilfsstoff enthält.

**16.** Verwendung einer Verbindung der Formel (I) nach einem der Ansprüche 1 bis 9 zur Herstellung eines Arzneimittels zur Behandlung und Prävention von kognitiven Störungen; Aufmerksamkeitsstörungen; Störungen der exekutiven Funktionen in Verbindung mit Alzheimer-Krankheit, pathologischer oder normaler Alterung, Parkinson-Syndrom, Trisomie 21, psychiatrischen Pathologien, Korsakoff-Alkoholsyndrom, vaskulären Demenzen oder Schädeltraumen; motorischen Störungen, die bei Parkinson-Krankheit oder anderen neurologischen Erkrankungen beobachtet werden, oder anatomisch-histopathologischen Störungen in Verbindung mit den oben aufgeführten neurodegenerativen Erkrankungen.

**17.** Verwendung einer Verbindung der Formel (I) nach einem der Ansprüche 1 bis 9 zur Herstellung eines Arzneimittels zur Behandlung und Prävention von Schlaganfällen, zerebralen hypoxischen Episoden und psychiatrischen Pathologien.

**18.** Verwendung einer Verbindung der Formel (I) nach einem der Ansprüche 1 bis 9 zur Herstellung eines Arzneimittels zur Prävention von durch Entzug von Tabak, Alkohol oder verschiedenen abhängigmachenden Substanzen verursachten Symptomen.

**19.** Verwendung einer Verbindung der Formel (I) nach einem der Ansprüche 1 bis 9 zur Herstellung eines Arzneimittels zur Behandlung von Schmerzen.

**20.** Verwendung einer Verbindung der Formel (I) nach einem der Ansprüche 1 bis 9 zur Herstellung eines Arzneimittels zur Behandlung von Ischämie der unteren Gliedmaßen, Arteritis obliterans der unteren Gliedmaßen, Herzischämie, Myokardinfarkt, Herzinsuffizienz, Hautvernarbungsdefizit bei Diabetespatienten und Ulcus varicosum bei Veneninsuffizienz.

**21.** Verwendung einer Verbindung der Formel (I) nach einem der Ansprüche 1 bis 9 zur Herstellung eines Arzneimittels zur Behandlung von Entzündungsprozessen.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 03057697 A
- WO 0305769 A


### Littérature non-brevet citée dans la description


- **Green et al.** Protective Groups in Organic Synthesis. John Wiley & Sons, Inc, 1991
- **J. March.** Advances in Organic Chemistry. Wiley Interscience, 1985, 310-316
- **Mark ; Collins.** *J. Pharmacol. Exp. Ther.,* 1982, vol. 22, 564
- **Marks.** *Mol. Pharmacol.,* 1986, vol. 30, 427
- **Anderson ; Americ.** *Eur. J. Pharmacol.,* 1994, vol. 253, 261
- **Hall.** *Brain Res.,* 1993, vol. 600, 127
- **Houghtling.** *Mol. Pharmacol.,* 1995, vol. 48, 280